(19) Europäisches Patentamt
European Patent Office
Office européen des brevets

(11) EP 0 908 456 B1

(12) **EUROPÄISCHE PATENTSCHRIFT**

(45) Veröffentlichungstag und Bekanntmachung des Hinweises auf die Patenterteilung:
**17.12.2003 Patentblatt 2003/51**

(51) Int Cl.[7]: **C07D 405/04**, A61K 31/415 // (C07D405/04, 307:00, 231:00)

(21) Anmeldenummer: **98118494.8**

(22) Anmeldetag: **30.09.1998**

(54) **Pyrazol-Derivate, ihre Herstellung und ihre Verwendung in Arzneimitteln**

Pyrazole derivatives, their preparation and their use in drugs

Dérivés de pyrazole, leur préparation et leur utilisation dans des médicaments

(84) Benannte Vertragsstaaten:
**AT BE CH CY DE DK ES FI FR GB GR IE IT LI LU NL PT SE**

(30) Priorität: **06.10.1997 DE 19744026**

(43) Veröffentlichungstag der Anmeldung:
**14.04.1999 Patentblatt 1999/15**

(73) Patentinhaber: **Aventis Pharma Deutschland GmbH**
**65929 Frankfurt am Main (DE)**

(72) Erfinder:
- **Schindler, Ursula, Dr.**
**65812 Bad Soden (DE)**
- **Schönafinger, Karl, Dr.**
**63755 Alzenau (DE)**
- **Strobel, Hartmut, Dr.**
**65835 Liederbach (DE)**

(56) Entgegenhaltungen:
**EP-A- 0 667 345**
**WO-A-98/16507**
**US-A- 4 072 498**

- **CHEMICAL ABSTRACTS, vol. 125, no. 3, 15. Juli 1996 Columbus, Ohio, US; abstract no. 33633, Seite 903; XP002056085 & CN 111 926 A (YONGXIN PHARMCEUTICAL INDUSTRY)**
- **CHEMICAL ABSTRACTS, vol. 84, no. 25, 21. Juni 1976 Columbus, Ohio, US; abstract no. 180207, Seite 575; XP002089978 & JP 50 121271 A (JAPAN)**
- **YU S -M ET AL: "INHIBITION OF PLATELET FUNCTION BY A02131-1, A NOVEL INHIBITOR OF CGMP-SPECIFIC PHOSPHODIESTERASE, IN VITRO AND IN VIVO" BLOOD, Bd. 87, Nr. 9, 1. Mai 1996, Seiten 3758-3767, XP002056082**
- **WU CH -CH ET AL: "GAMMAC-1 INHIBITED HUMAN PLATELET AGGREGATION THROUGH NO-INDEPENDENT ACTIVATION OF SOLUBLE GUANYLATE CYCLASE" BRITISH JOURNAL OF PHARMACOLOGY, Bd. 116, Nr. 3, 1995, Seiten 1973-1978, XP002056083**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 908 456 B1

Printed by Jouve, 75001 PARIS (FR)

## Beschreibung

Die vorliegende Erfindung betrifft Pyrazol-Derivate der Formel 1,

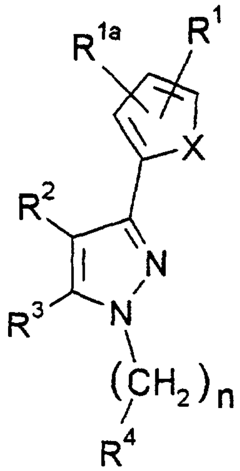

I

in der X, $R^1$, $R^{1a}$, $R^2$, $R^3$, $R^4$ und n die unten angegebenen Bedeutungen haben, die wertvolle Arzneimittelwirkstoffe zur Therapie und Prophylaxe von Krankheiten sind, zum Beispiel von Herz-Kreislauferkrankungen wie Bluthochdruck, Angina pectoris, Herzinsuffizienz, Thrombosen oder Atherosklerose. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind. Die Erfindung betrifft weiterhin Verfahren zur Herstellung von Verbindungen der Formel I, ihre Verwendung zur Therapie und Prophylaxe der bezeichneten Krankheitszustände und zur Herstellung von Arzneimitteln dafür, sowie pharmazeutische Präparate, die Verbindungen der Formel I enthalten.

cGMP ist ein wichtiger intrazellulärer Botenstoff, der über die Modulation cGMP-abhängiger Proteinkinasen, Phosphodiesterasen und Ionenkanälen eine Vielzahl verschiedener Effekte auslöst. Beispiele sind die Glattmuskelrelaxation, die Inhibition der Thrombozytenaktivierung und die Hemmung der Glattmuskelzellproliferation und der Leukozytenadhäsion. cGMP wird durch partikuläre und lösliche Guanylatcyclasen (GC) als Antwort auf eine Reihe extraund intrazellulärer Stimuli produziert. Im Falle der partikulären Guanylatcyclasen erfolgt die Stimulation im wesentlichen durch peptidische Signalstoffe, wie dem atrialen natriuretischen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies. Die Bedeutung anderer Faktoren wie Kohlenmonoxid oder dem Hydroxylradikal ist noch weitgehend ungeklärt. Als Aktivierungsmechanismus der Aktivierung durch NO wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins überführt das Enzym in die aktivierte Konformation.

Aktive lösliche Guanylatcyclasen setzen sich aus je einer $\alpha$- und einer $\beta$-Untereinheit zusammen. Von den Untereinheiten wurden mehrere Subtypen beschrieben, die sich untereinander bezüglich Sequenz, gewebespezifischer Verteilung und Expression in verschiedenen Entwicklungsstadien unterscheiden. Die Subtypen $\alpha_1$ und $\beta_1$ werden hauptsächlich in Gehirn und Lunge exprimiert, während $\beta_2$ vor allem in Leber und Niere gefunden wird. In humanem fötalen Gehirn konnte der Subtyp $\alpha_2$ nachgewiesen werden. Die als $\alpha_3$ und $\beta_3$ bezeichneten Untereinheiten wurden aus menschlichem Gehirn isoliert und sind homolog zu $\alpha_1$ und $\beta_1$. Neuere Arbeiten weisen auf eine $\alpha_{2i}$-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über $\beta_1$-Cys-78 und/oder $\beta_1$-His-105 gebunden ist und Teil des regulatorischen Zentrums ist.

Unter pathologischen Bedingungen kann die Bildung Guanylatcyclase-aktivierender Faktoren vermindert sein oder es kann durch das vermehrte Auftreten freier Radikale ein verstärkter Abbau derselben erfolgen. Die daraus resultierende verminderte Aktivierung der sGC führt über die Abschwächung der jeweiligen cGMP-vermittelten Zellantwort beispielsweise zum Anstieg des Blutdrucks, zur Plättchenaktivierung oder zu vermehrter Zellproliferation und Zelladhäsion. Als Folge kommt es zur Ausbildung von endothelialer Dysfunktion, Atherosklerose, Bluthochdruck, stabiler und instabiler Angina pectoris, Thrombosen, Myocardinfarkt, Schlaganfällen oder erektiler Dysfunktion. Die pharmakologische Stimulation der sGC bietet eine Möglichkeit zur Normalisierung der cGMP-Produktion und erlaubt damit die Behandlung bzw. Prävention derartiger Krankheiten.

Zur pharmakologischen Stimulation der sGC wurden bisher fast ausschließlich Verbindungen verwendet, deren Wirkung auf einer intermediären NO-Freisetzung beruht, beispielsweise organische Nitrate. Der Nachteil dieser Behandlungsweise liegt in der Toleranzentwicklung und Wirkungsabschwächung und der deshalb erforderlich werdenden höheren Dosierung.

Verschiedene nicht über eine NO-Freisetzung wirkende sGC-Stimulatoren wurden von Veseley in einer größeren Zahl von Arbeiten beschrieben. Die Verbindungen, bei denen es sich zumeist um Hormone, Pflanzenhormone, Vitamine oder zum Beispiel Naturstoffe wie Echsengifte handelt, zeigen jedoch durchweg nur schwache Effekte auf die cGMP-Bildung in Zellysaten (D. L. Veseley, Eur. J. Clin. Invest. 15 (1985) 258; D. L. Veseley, Biochem. Biophys. Res. Comm. 88 (1979) 1244). Eine Stimulation Häm-freier Guanylatcyclase durch Protoporphyrin IX wurde durch Ignarro et al. (Adv. Pharmacol. 26 (1994) 35) nachgewiesen. Pettibone et al. (Eur. J. Pharmacol. 116 (1985) 307) beschrieben für Diphenyliodoniumhexafluorophosphat eine blutdrucksenkende Wirkung und führten diese auf eine Stimulation der sGC zurück. Isoliquiritiginin, das an isolierten Rattenaorten eine relaxierende Wirkung zeigt, aktiviert laut Yu et al. (Brit. J. Pharmacol. 114 (1995) 1587) ebenfalls die sGC. Ko et al. (Blood 84 (1994) 4226), Yu et al. (Biochem. J. 306 (1995) 787) und Teng et al. (Brit. J. Pharmacol. 116 (1995) 1973) wiesen eine sGC-stimulierende Aktivität von 1-Benzyl-3-(5-hydroxymethyl-2-furyl)-indazol nach und demonstrierten eine antiproliferative und thrombozytenhemmende Wirkung. Verschiedene Indazole werden in EP-A-667 345 als Inhibitoren der Thrombozytenaggregation beschrieben.

Überraschend wurde nun gefunden, daß die Pyrazol-Derivate der Formel I eine Guanylatcyclase-Aktivierung bewirken und damit für die Therapie und Prophylaxe von Krankheiten geeignet sind, die mit einem niedrigen cGMP-Spiegel verbunden sind.

Die vorliegende Erfindung betrifft somit Verbindungen der Formel I,

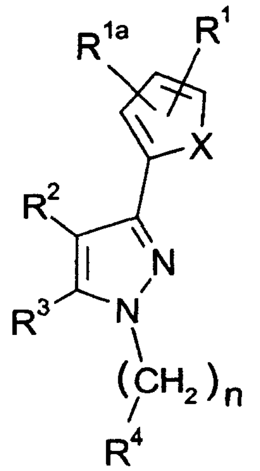

I

in der

X für O, S, NH oder $N(CH_3)$ steht;

$R^1$ für $CO-NR^{11}R^{12}$ steht;

$R^{1a}$ für Wasserstoff steht;

$R^2$ und $R^3$ unabhängig voneinander für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het oder den Rest Het-$(C_1-C_4)$-alkyl stehen, wobei für $R^2$ oder $R^3$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere Substituenten $R^5$ substituiert sein können,

oder $R^2$ und $R^3$ zusammen mit den sie tragenden Kohlenstoffatomen einen 5- bis 7-gliedrigen carbocyclischen Ring bilden, der eine oder mehrere Doppelbindungen enthalten kann und der unsubstituiert oder durch einen oder mehrere Substituenten $R^5$ substituiert sein kann;

$R^4$ für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het oder den Rest Het-$(C_1-C_4)$-alkyl stehen, wobei für $R^4$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere Substituenten $R^5$ substituiert sein können, und wobei, wenn n = 0 ist, $R^4$ nicht für Wasserstoff stehen kann;

n für 0, 1 oder 2 steht;
Het für einen 5- bis 7-gliedrigen, gesättigten oder ungesättigten Heterocyclus steht;
$R^5$ für Halogen, $(C_1-C_5)$-Alkyl, $OR^6$, $NR^7R^8$, $CO-OR^9$, $CO-R^{10}$, $CO-NR^{11}R^{12}$, $CO-NR^{12}-OR^{11}$, $S(O)_m-R^{13}$, $S(O)_2-NR^{14}R^{15}$, $NO_2$, CN oder $CF_3$ steht, wobei mehrfach auftretende Reste $R^5$ unabhängig voneinander sind und gleich oder verschieden sein können;
$R^6$, $R^7$, $R^8$, $R^{11}$ und $R^{14}$ unabhängig voneinander für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het, den Rest Het-$(C_1-C_4)$-alkyl, $CO-R^{16}$ oder $S(O)_2-R^{17}$ stehen, wobei für $R^6$, $R^7$, $R^8$, $R^{11}$ und $R^{14}$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere gleiche oder verschiedene Substituenten aus der Reihe Halogen, $(C_1-C_5)$-Alkyl, $OR^{18}$, $NR^{19}R^{20}$, $CO-OR^{21}$, $CO-R^{22}$, $CO-NR^{23}R^{24}$, $CO-NR^{24}-OR^{23}$, $S(O)_m-R^{25}$, $S(O)_2-NR^{26}R^{27}$, $NO_2$, CN und $CF_3$ substituiert sein können, und wobei mehrfach auftretende Reste $R^6$, $R^7$, $R^8$, $R^{11}$ und $R^{14}$ unabhängig voneinander sind und gleich oder verschieden sein können;
$R^9$, $R^{10}$, $R^{12}$, $R^{13}$ und $R^{15}$ unabhängig voneinander für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het oder den Rest Het-$(C_1-C_4)$-alkyl stehen, wobei für $R^9$, $R^{10}$, $R^{12}$, $R^{13}$ und $R^{15}$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere gleiche oder verschiedene Substituenten aus der Reihe Halogen, $(C_1-C_5)$-Alkyl, $OR^{18}$, $NR^{19}R^{20}$, $CO-OR^{21}$, $CO-R^{22}$, $CO-NR^{23}R^{24}$, $CO-NR^{24}-OR^{23}$, $S(O)_m-R^{25}$, $S(O)_2$ $NR^{26}R^{27}$, $NO_2$, CN und $CF_3$ substituiert sein können, und wobei mehrfach auftretende Reste $R^9$, $R^{10}$, $R^{12}$, $R^{13}$ und $R^{15}$ unabhängig voneinander sind und gleich oder verschieden sein können;
oder die zwei Reste $R^7$ und $R^8$, die zwei Reste $R^{11}$ und $R^{12}$ und die zwei Reste $R^{14}$ und $R^{15}$, jeweils zusammen mit dem die zwei Reste tragenden Stickstoffatom, einen 5- bis 7-gliedrigen, gesättigten oder ungesättigten heterocyclischen Ring bilden, der noch ein zusätzliches Ring-Heteroatom aus der Reihe Stickstoff, Sauerstoff und Schwefel enthalten kann und der durch einen oder mehrere gleiche oder verschiedene Substituenten aus der Reihe $(C_1-C_4)$-Alkyl und Halogen substituiert sein kann;
$R^{16}$ für Wasserstoff, $(C_1-C_6)$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het oder den Rest Het-$(C_1-C_4)$-alkyl steht;
$R^{17}$ für $(C_1-C_6)$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het oder den Rest Het-$(C_1-C_4)$-alkyl steht;
$R^{18}$, $R^{19}$, $R^{20}$, $R^{23}$ und $R^{26}$ unabhängig voneinander für Wasserstoff, $(C_1-C_{10})$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het, den Rest Het-$(C_1-C_4)$-alkyl, $CO-R^{16}$ oder $S(O)_2-R^{17}$ stehen, wobei mehrfach auftretende Reste $R^{18}$, $R^{19}$, $R^{20}$, $R^{23}$ und $R^{26}$ unabhängig voneinander sind und gleich oder verschieden sein können;
$R^{21}$, $R^{22}$, $R^{24}$, $R^{25}$ und $R^{27}$ unabhängig voneinander für Wasserstoff, $(C_1-C_{10})$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het oder den Rest Het-$(C_1-C_4)$-alkyl stehen, wobei mehrfach auftretende Reste $R^{21}$, $R^{22}$, $R^{24}$, $R^{25}$ und $R^{27}$ unabhängig voneinander sind und gleich oder verschieden sein können;
oder die zwei Reste $R^{19}$ und $R^{20}$, die zwei Reste $R^{23}$ und $R^{24}$ und die zwei Reste $R^{26}$ und $R^{27}$, jeweils zusammen mit dem die zwei Reste tragenden Stickstoffatom, einen 5- bis 7-gliedrigen, gesättigten oder ungesättigten heterocyclischen Ring bilden, der noch ein zusätzliches Ring-Heteroatom aus der Reihe Stickstoff, Sauerstoff und Schwefel enthalten kann und der durch einen oder mehrere gleiche oder verschiedene Substituenten aus der Reihe $(C_1-C_4)$-Alkyl und Halogen substituiert sein kann;
m für 0, 1 oder 2 steht;

in allen ihren stereoisomeren Formen und Mischungen davon in allen Verhältnissen, und ihre physiologisch verträglichen Salze.

Alkylreste können geradkettig oder verzweigt sein. Dies gilt auch, wenn sie substituiert sind, zum Beispiel durch einen Arylrest in einer Arylalkylgruppe oder durch den Rest Het in der Gruppe Het-alkyl, oder wenn sie in anderen Gruppen enthalten sind, zum Beispiel in Alkoxygruppen, Alkoxycarbonylgruppen oder N-alkylsubstituierten Carbamoylgruppen. Beispiele für Alkylgruppen sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, n-Heptyl, n-Octyl, n-Nonyl und n-Decyl. Unter dem Begriff Alkyl sind hier auch ungesättigte Alkylreste zu verstehen, insbesondere Alkylreste, die eine oder zwei Doppelbindungen oder eine oder zwei Dreifachbindungen oder eine Doppelbindung und eine Dreifachbindung enthalten. Beispiele für solche Reste sind der Vinylrest, der 2-Propenylrest (Allylrest), der 2-Butenylrest, der 3-Methyl-2-butenylrest, der Ethinylrest, der 2-Propinylrest (Propargylrest) oder der 3-Butinylrest.

Beispiele für Cycloalkylreste sind Cyclopropyl, Cyclobutyl, Cyclopentyl, Cyclohexyl und Cycloheptyl. Cycloalkylreste können auch durch Alkylgruppen, zum Beispiel durch eine oder mehrere Methylgruppen, substituiert sein.

Beispiele für $(C_6-C_{14})$-Arylreste sind Phenyl, Naphthyl, Anthracenyl, Biphenylyl oder Fluorenyl, wobei die mehrkemigen Reste über alle Positionen verknüpft sein können. Naphthylreste zum Beispiel können also als 1-Naphthylreste oder 2-Naphthylreste vorliegen. Ein bevorzugter Arylrest ist der Phenylrest. Arylreste können unsubstituiert

oder einfach oder mehrfach, zum Beispiel zweifach oder dreifach, substituiert sein, wobei sich die Substituenten in beliebigen Positionen befinden können. Monosubstituierte Phenylreste können in der 2-Position, der 3-Position oder der 4-Position substituiert sein, disubstituierte Phenylreste in der 2,3-Position, der 2,4-Position, der 2,5-Position, der 2,6-Position, der 3,4-Position oder der 3,5-Position. In trisubstituierten Phenylresten können sich die Substituenten beispielsweise in 2,3,4-Position, 2,3,5-Position, 2,3,6-Position oder 3,4,5-Position befinden. Diese Erläuterungen zu Arylresten gelten auch für solche Arylreste, die in Arylalkylresten enthalten sind, beispielsweise in Benzylresten, 1-Phenylethylresten, 2-Phenylethylresten oder Naphthylmethylresten. Bevorzugte Arylalkylreste sind Phenylethylreste und insbesondere der Benzylrest.

Die für die Gruppe Het stehenden 5- bis 7-gliedrigen, gesättigten oder ungesättigten Heterocyclen enthalten bevorzugt ein, zwei, drei oder vier Ring-Heteroatome aus der Reihe Stickstoff, Sauerstoff und Schwefel. Besonders bevorzugt enthalten sie ein, zwei oder drei Heteroatome aus dieser Reihe. Ungesättigte Heterocyclen können eine, zwei oder drei Doppelbindungen im Ring enthalten. Die 5-Ring- und 6-Ring-Heterocyclen können insbesondere auch aromatisch sein, daß heißt, Het kann in den Verbindungen der Formel I auch für 5- oder 6-gliedriges Heteroaryl stehen, daß unsubstituiert oder substituiert sein kann. Beispiele für heterocyclische 5-Ring-, 6-Ring- und 7-Ring-Systeme, von denen sich in Betracht kommende Reste ableiten können, sind Pyrrol, Furan, Thiophen, Imidazol, Pyrazol, 1,2,3-Triazol, 1,2,4-Triazol, 1,3-Dioxol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxepin oder 1,3-Thiazepin, alle jeweils in gesättigter Form (Perhydro-Form) oder in teilweise ungesättigter Form (Dihydro-Form und Tetrahydro-Form) oder in maximal ungesättigter Form (aromatischer Form im Falle der 5-Ringe und 6-Ringe), soweit die betreffenden Formen bekannt und stabil sind. Zu den in Betracht kommenden Heterocyclen gehören also auch beispielsweise die gesättigten Heterocyclen Pyrrolidin, Piperidin, Piperazin, Morpholin und Thiomorpholin. Der heterocyclische Rest kann über ein beliebiges Kohlenstoffatom gebunden sein, er kann also zum Beispiel im Fall von Resten, die sich vom Furansystem, dem Thiophensystem oder dem Pyrrolsystem ableiten, in der 2-Position oder der 3-Position gebunden sein, im Fall von Resten, die sich vom Imidazolsystem ableiten oder vom 1,3-Thiazolsystem ableiten, in der 2- Position, der 4-Position oder der 5-Position, oder im Fall von Resten, die sich vom Pyridinsystem ableiten, in der 2-Position, der 3-Position oder der 4-Position. Stickstoffheterocyclen, die an einem Ring-Stickstoffatom einen Substituenten tragen können, können auch über ein Ring-Stickstoffatom gebunden sein, wenn der betreffende heterocyclische Rest an ein Kohlenstoffatom gebunden ist. Die Heterocyclen können einfach oder mehrfach, zum Beispiel zweifach, dreifach oder vierfach, und in beliebigen Positionen substituiert sein. Substituenten an einem Heterocyclus können auch einen Ring bilden, es können also kondensierte Heterocyclen vorliegen, zum Beispiel cyclopenta-kondensierte, cyclohexa-kondensierte oder benzo-kondensierte Heterocyclen. Als Substituenten an einem Stickstoffatom eines Heterocyclus kommen insbesondere zum Beispiel $(C_1\text{-}C_5)$-Alkylreste oder die Reste $CO\text{-}R^{16}$ oder $SO_2\text{-}R^{17}$ in Betracht, aber auch zum Beispiel Arylreste oder Arylalkylreste. Stickstoffheterocyclen können auch als N-Oxide vorliegen.

Halogen bedeutet, sofern nicht anders angegeben, Fluor, Chlor, Brom oder Iod, vorzugsweise Fluor oder Chlor.

Sind Alkylreste, Arylreste, Arylalkylreste, Cycloalkylreste, Cycloalkyl-alkylreste oder die Reste Het oder Het-alkyl durch Reste $R^5$ substituiert, so können sie durch einen, zwei, drei, vier oder mehr gleiche oder verschiedene Reste $R^5$ substituiert sein. Bevorzugt enthalten solche substituierten Reste einen, zwei oder drei Reste gleiche oder verschiedene Reste $R^5$. Die Reste $R^5$ können sich im Falle der Arylalkylreste, Cycloalkyl-alkylreste und der Reste Het-alkyl jeweils im Alkylteil und/oder im Arylteil bzw. Cycloalkylteil bzw. dem Rest Het befinden. Wenn $R^2$ und $R^3$ zusammen mit den sie tragenden Kohlenstoffatomen einen carbocyclischen Ring bilden, der durch einen oder mehrere Reste $R^5$ substituiert ist, so ist er bevorzugt durch einen, zwei, drei oder vier gleiche oder verschiedene Reste $R^5$ substituiert, besonders bevorzugt durch einen oder zwei Reste. Die Reste $R^5$ können sich in beliebigen Positionen befinden.

Die beiden Substituenten $R^1$ und $R^{1a}$ können sich in beliebigen Positionen des Heterocyclus befinden, also in den Positionen 3, 4 und 5 des Furanrings, Thiophenrings oder Pyrrolrings.

Bilden $R^2$ und $R^3$ zusammen mit den sie tragenden Kohlenstoffatomen einen 5- bis 7-gliedrigen carbocyclischen Ring, so liegen kondensierte Pyrazole vor. Im Falle eines ankondensierten 5-Rings, also im Falle von cyclopenta-kondensierten Pyrazolen, kann der carbocyclische Ring neben der Doppelbindung, die er mit dem Pyrazolring gemeinsam hat, noch eine weitere Doppelbindung im Ring enthalten oder keine weitere Doppelbindung im Ring enthalten. Im Falle eines ankondensierten 6-Rings, also im Falle von cyclohexa-kondensierten Pyrazolen, oder im Falle eines ankondensierten 7-Rings, also im Falle eines cycloheptakondensierten Pyrazols, kann der carbocyclische Ring neben der Doppelbindung, die er mit dem Pyrazolring gemeinsam hat, noch eine weitere Doppelbindung im Ring oder zwei weitere Doppelbindungen im Ring enthalten oder keine weitere Doppelbindungen im Ring enthalten. Die Doppelbindungen im carbocyclischen Ring können sich in beliebigen Positionen befinden, es können aber keine kumulierten Doppelbindungssysteme vorliegen. Bilden $R^2$ und $R^3$ zusammen mit den sie tragenden Kohlenstoffatomen einen 6-gliedrigen Carbocyclus mit insgesamt drei Doppelbindungen, so ist ein Benzolring an den Pyrazolring ankondensiert,

bei den Verbindungen der Formel I handelt es sich in diesem Fall dann um Benzo-Pyrazole (Formel Ia), die auch als Indazole bezeichnet werden (die erfindungsgemäßen Verbindungen sind 1 H-Indazole, so wie auch die nicht benzokondensierten Pyrazole 1 H-Pyrazole sind). Beispiele für derartige Verbindungen der Formel I, in der $R^2$ und $R^3$ zusammen mit den sie tragenden Kohlenstoffatomen einen carbocyclischen Ring bilden, sind die Verbindungen der Formeln Ia, Ib, Ic und Id, in denen X, $R^1$, $R^{1a}$, $R^4$, $R^5$ und n die oben angegebenen Bedeutungen haben und y entsprechend den obigen Angaben für 0 oder eine ganze Zahl, bevorzugt für 0, 1, 2, 3 oder 4 steht, besonders bevorzugt für 0, 1 oder 2 steht. Die Reste $R^5$, die gleich oder verschieden sein können, können sich in beliebigen Positionen im Carbocyclus befinden.

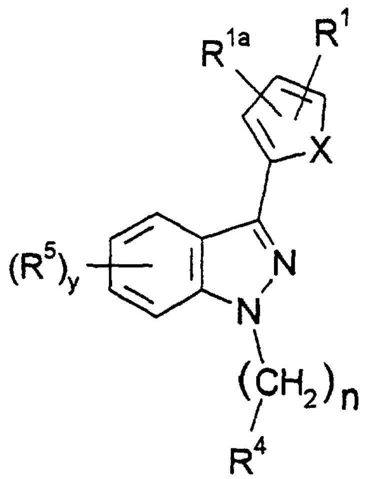
Ia

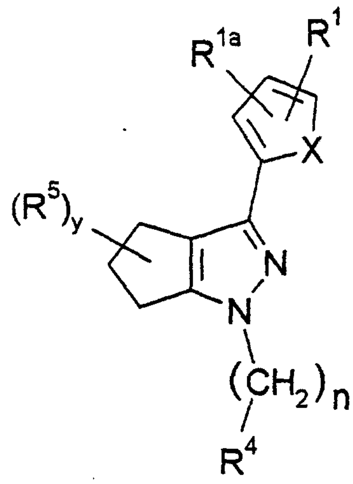
Ib

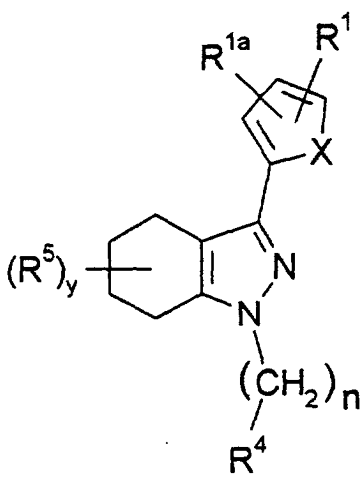
Ic

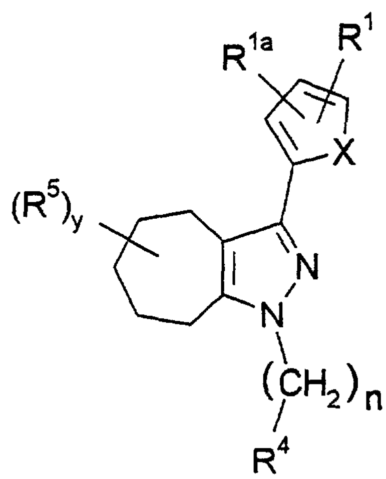
Id

Die heterocyclischen Ringe, die die zwei Reste $R^7$ und $R^8$, die zwei Reste $R^{11}$ und $R^{12}$, die zwei Reste $R^{14}$ und $R^{15}$, die zwei Reste $R^{19}$ und $R^{20}$, die zwei Reste $R^{23}$ und $R^{24}$ und die zwei Reste $R^{26}$ und $R^{27}$, jeweils zusammen mit dem die zwei Reste tragenden Stickstoffatom, bilden können, können gesättigt oder teilweise ungesättigt oder maximal ungesättigt sein. Bevorzugt sind sie gesättigt. Beispiele für solche Ringe, die über ein Ring-Stickstoffatom an eine Carbonylgruppe oder eine Sulfonylgruppe gebunden sind, sind insbesondere Pyrrolin, Pyrrolidin, Piperidin, Piperazin, Morpholin und Thiomorpholin. Diese Ringe können an Kohlenstoffatomen und im Falle des Piperazins auch am

Stickstoffatom in der 4-Position substituiert sein und im Falle des Thiomorpholins auch am Schwefel zum Sulfoxid oder zum Sulfon oxidiert sein.

Steht n in der Formel I für die Zahl 0, so ist der Rest $R^4$ direkt an den das Pyrazol-Stickstoffatom gebunden.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als links- als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Die Trennung eines Stereoisomerengemisches kann auf der Stufe der Verbindungen der Formel I erfolgen oder im Verlaufe der Synthese.

Bei Vorliegen von beweglichen Wasserstoffatomen umfaßt die vorliegende Erfindung auch alle tautomeren Formen der Verbindungen der Formel I, zum Beispiel Lactam/Lactim-Tautomere.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die eine oder mehrere saure Gruppen enthalten, zum Beispiel COOH-Gruppen oder N-Acylsulfonamidgruppen, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und Metabolite, die wie die Verbindungen der Formel I wirken.

In der Formel I steht X bevorzugt für O oder S, besonders bevorzugt für O.

Bevorzugt befindet sich der Rest $R^1$ in der 5-Position des Heterocyclus.

$R^2$ und $R^3$ bilden bevorzugt zusammen mit den sie tragenden Kohlenstoffatomen einen Benzolring, der unsubstituiert oder durch einen oder mehrere gleiche oder verschiedene Substituenten $R^5$ substituiert sein kann (bei den erfindungsgemäßen Verbindungen, in denen diese bevorzugten Bedeutungen von $R^2$ und $R^3$ vorliegen, handelt es sich um die benzo-kondensierten Pyrazole der Formel Ia).

n steht bevorzugt für 0 oder 1.

$R^4$ steht bevorzugt für ($C_6$-$C_{14}$)-Aryl, insbesondere Phenyl, oder 5- oder 6-gliedriges Heteroaryl, wobei diese Reste substituiert oder unsubstituiert sein können.

In den Resten $R^2$ und $R^3$ vorkommende Reste $R^5$ stehen bevorzugt für Halogen, ($C_1$-$C_3$)-Alkyl, $CF_3$, ($C_1$-$C_3$)-Alkyl-O oder $S(O)_2$-$NR^{14}R^{15}$, besonders bevorzugt für $CF_3$, im Rest $R^4$ vorkommende Reste $R^5$ stehen bevorzugt für Halogen, ($C_1$-$C_3$)-Alkyl, oder $CF_3$.

$R^6$ steht bevorzugt für Wasserstoff oder ($C_1$-$C_3$)-Alkyl.

$R^7$ steht bevorzugt für Wasserstoff, ($C_1$-$C_3$)-Alkyl, CO-$R^{16}$ oder $S(O)_2$-$R^{17}$.

$R^8$ steht bevorzugt für Wasserstoff.

$R^9$ steht bevorzugt für $CH_2CH_2$-OH oder $CH_2CH_2$-$NR^{19}R^{20}$.
$R^{10}$ steht bevorzugt für ($C_1$-$C_3$)-Alkyl oder unsubstituiertes oder substituiertes Phenyl.
$R^{11}$ steht bevorzugt für Wasserstoff.
$R^{12}$ steht bevorzugt für unsubstituiertes ($C_1$-$C_4$)-Alkyl, ($C_1$-$C_4$)-Alkyl, das durch einen Rest aus der Reihe $OR^{18}$, $NR^{19}R^{20}$ und CO-$NR^{23}R^{24}$ substituiert ist, 5- oder 6-gliedriges Heteroaryl, unsubstituiertes Phenyl oder Phenyl, das durch einen, zwei oder drei gleiche oder verschiedene Reste aus der Reihe Halogen, ($C_1$-$C_5$)-Alkyl, $OR^{18}$, $NR^{19}R^{20}$, CO-$OR^{21}$, CO-$R^{22}$, CO-$NR^{23}R^{24}$, $S(O)_m$-$R^{25}$, $S(O)_2$-$NR^{26}R^{27}$, $NO_2$, CN und $CF_3$ substituiert ist.
$R^{13}$ steht bevorzugt für ($C_1$-$C_3$)-Alkyl, unsubstituiertes Phenyl, Phenyl, das durch einen, zwei oder drei gleiche oder verschiedene Reste aus der Reihe Halogen, ($C_1$-$C_5$)-Alkyl, $OR^{18}$, $NR^{19}R^{20}$, CO-$OR^{21}$, CO-$R^{22}$, CO-$NR^{23}R^{24}$, $S(O)_m$-$R^{25}$, $S(O)_2$-$NR^{26}R^{27}$, $NO_2$, CN und $CF_3$ substituiert ist, oder für 5- oder 6-gliedriges Heteroaryl.
$R^{14}$ steht bevorzugt für ($C_1$-$C_3$)-Alkyl, 5- oder 6-gliedriges Heteroaryl, CO-$R^{16}$, unsubstituiertes Phenyl oder Phenyl, das durch einen, zwei oder drei gleiche oder verschiedene Reste aus der Reihe Halogen, ($C_1$-$C_5$)-Alkyl, $OR^{18}$, $NR^{19}R^{20}$, CO-$OR^{21}$, CO-$R^{22}$, CO-$NR^{23}R^{24}$, $S(O)_m$-$R^{25}$, $S(O)_2$-$NR^{26}R^{27}$, $NO_2$, CN und $CF_3$ substituiert ist;
$R^{15}$ steht bevorzugt für Wasserstoff.

Ebenfalls bevorzugt ist es, wenn die Reste $R^{14}$ und $R^{15}$ zusammen mit dem die zwei Reste tragenden Stickstoffatom einen 5- bis 7-gliedrigen gesättigen Ring bilden, der besonders bevorzugt als zusätzliches Ring-Heteroatom ein Sauerstoffatom, ein Schwefelatom oder ein durch eine Methylgruppe substituiertes Stickstoffatom enthält.
$R^{16}$ steht bevorzugt für ($C_1$-$C_3$)-Alkyl.
$R^{17}$ steht bevorzugt für ($C_1$-$C_3$)-Alkyl.
$R^{18}$ steht bevorzugt für Wasserstoff oder ($C_1$-$C_3$)-Alkyl.
$R^{19}$ steht bevorzugt für Wasserstoff.
$R^{20}$ steht bevorzugt für CO-($C_1$-$C_3$)-Alkyl oder CO-($C_6$-$C_{14}$)-Aryl, $S(O)_2$-($C_1$-$C_3$)-Alkyl oder $S(O)_2$-($C_6$-$C_{14}$)-Aryl.
$R^{21}$, $R^{22}$, $R^{24}$ und $R^{25}$ stehen bevorzugt für ($C_1$-$C_3$)-Alkyl.
$R^{23}$ steht bevorzugt für Wasserstoff oder ($C_1$-$C_3$)-Alkyl.
$R^{26}$ steht bevorzugt für ($C_1$-$C_3$)-Alkyl oder CO-$R^{16}$.
$R^{27}$ steht bevorzugt für Wasserstoff.

Ebenfalls bevorzugt ist es, wenn $R^{26}$ und $R^{27}$ zusammen mit dem die zwei Reste tragenden Stickstoffatom einen 5- bis 7-gliedrigen gesättigen Ring bilden, der besonders bevorzugt als zusätzliches Ring-Heteroatom ein Sauerstoffatom, ein Schwefelatom oder ein durch eine Methylgruppe substituiertes Stickstoffatom enthält.

Ein 5- oder 6-gliedriger Heteroarylrest bedeutet bevorzugt den Rest eines der aromatischen Heterocyclus mit einem, zwei, drei oder vier Heteroatomen aus der Reihe Stickstoff, Sauerstoff und Schwefel, besonders bevorzugt mit einem oder zwei Heteroatomen oder den Tetrazolylrest. Ganz besonders bevorzugt bedeutet ein 5-oder 6-gliedriger Heteroarylrest den Rest eines der aromatischen Heterocyclen Furan, Thiophen, 1,3-Thiazol, 1,3-Oxazol, 1,2-Oxazol, Tetrazol, Pyridin und Pyrimidin, darüber hinaus bevorzugt des 1,3-Thiazols oder des Tetrazols. Diese Reste sind über ein Kohlenstoffatom gebunden sind und können unsubstituiert oder wie oben angegeben substituiert sein.

Bevorzugte Verbindungen der Formel I sind solche, in denen einer oder mehrere der darin enthaltenen Reste bevorzugte Bedeutungen haben, wobei alle Kombinationen von bevorzugten Substituentendefinitionen umfaßt werden. Auch von allen bevorzugten Verbindungen der Formel I umfaßt die vorliegende Erfindung alle ihre stereoisomeren Formen und Mischungen davon in allen Verhältnissen, und ihre physiologisch verträglichen Salze.

Die Verbindungen der Formel I können nach verschiedenen Verfahren hergestellt werden, die im folgenden beschrieben sind und die ebenfalls Gegenstand der vorliegenden Erfindung sind. Verbindungen der Formel I, in der die Reste $R^2$ und $R^3$ nicht zusammen mit den sie tragenden Kohlenstoffatomen einen Benzolring bilden, in der die Reste $R^2$ und $R^3$ also für Wasserstoff oder einen unsubstituierten oder substituierten Alkylrest, Arylrest, Arylalkylrest, Cycloalkylrest, Cyclalkyl-alkylrest, den Rest Het oder den Rest Het-alkyl stehen oder in der $R^2$ und $R^3$ zusammen mit den sie tragenden Kohlenstoffatomen einen unsubstituierten oder substituierten nichtaromatischen carbocyclischen Ring bilden, können hergestellt werden, indem man 1,3-Dicarbonylverbindungen der Formel II mit Hydrazinen der Formel III oder deren Salzen zu Verbindungen der Formel Ie' umsetzt. In den Formeln II und III und in der Formel Ie' können die Reste X, $R^{1'}$, $R^{1a'}$, $R^{2'}$, $R^{3'}$ und $R^{4'}$ und die Zahl n die oben angegebenen Bedeutungen von X, $R^1$, $R^{1a}$, $R^2$, $R^3$, $R^4$ und n haben, die Reste $R^{2'}$ und $R^{3'}$ aber nicht zusammen mit den sie tragenden Kohlenstoffatomen einen aromatischen Ring bilden können, und zusätzlich können in den Resten $R^{1'}$, $R^{1a'}$, $R^{2'}$, $R^{3'}$ und $R^{4'}$ funktionelle Gruppen in geschützter Form oder in Form von Vorstufen vorliegen. Geeignete Schutzgruppen bzw. günstige Vorstufen für funktionelle Gruppen in diesen Resten sind dem Fachmann bekannt. Beispielsweise kann eine Carbonylgruppe in diesen Resten zunächst in geschützter Form vorliegen, zum Beispiel in Form eines Acetals oder Ketals, oder es kann eine Aminogruppe in acylierter Form vorliegen oder es kann ein Wasserstoffatom als Vorstufe für eine in einer elektrophilen Substitutionsreaktion eingeführte Gruppe vorliegen. Aus den Verbindungen der Formel Ie' können dann gegebenenfalls erfindungsgemäße Verbindungen der Formel Ie erhalten werden, indem man in einem anschließenden Reaktionsschritt die in geschützter Form bzw. in Form von Vorstufen vorliegenden Gruppen in die gewünschten, in den obigen Definitionen von $R^1$, $R^{1a}$, $R^2$, $R^3$ und $R^4$ genannten funktionellen Gruppen überführt.

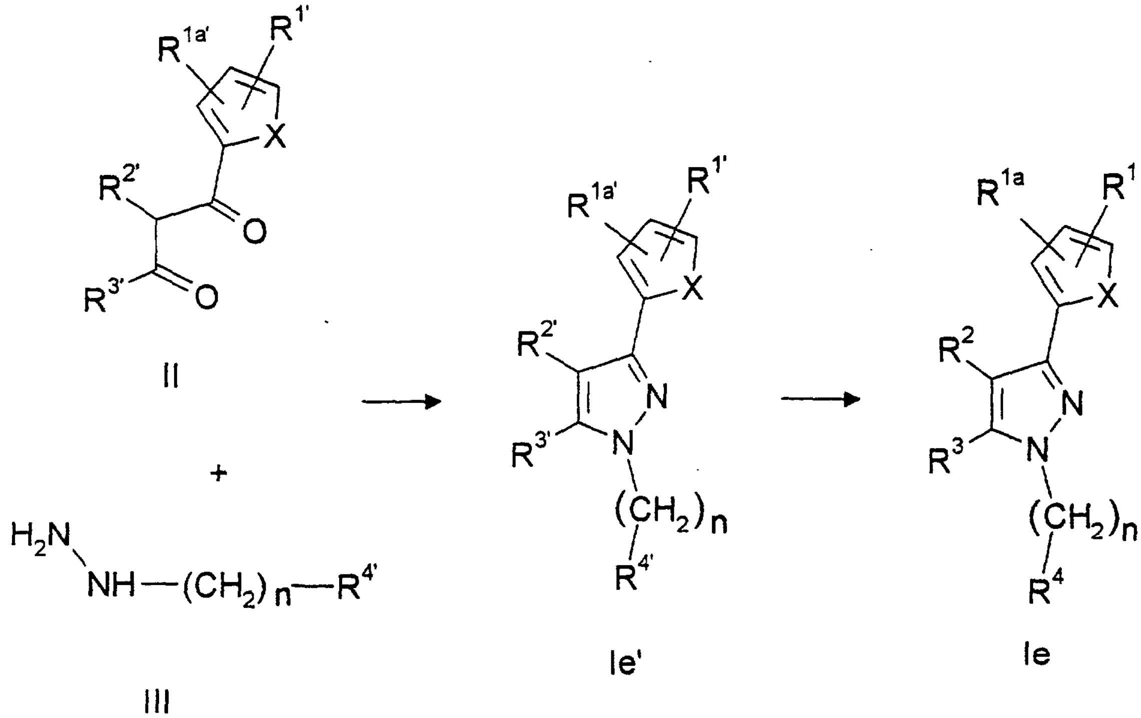

II + III → Ie' → Ie

In den so erhältlichen Verbindungen der Formel Ie haben also X, $R^1$, $R^{1a}$, $R^2$, $R^3$, $R^4$ und n die oben für die Formel I angegebenen Bedeutungen, $R^2$ und $R^3$ können aber nicht zusammen mit den sie tragenden Kohlenstoffatomen einen Benzolring bilden, $R^2$ und $R^3$ stehen also für Wasserstoff oder einen unsubstituierten oder substituierten Alkylrest, Arylrest, Arylalkylrest, Cycloalkylrest, Cycloalkyl-alkylrest, den Rest Het oder den Rest Het-alkyl oder $R^2$ und $R^3$ bilden zusammen mit den sie tragenden Kohlenstoffatomen einen unsubstituierten oder substituierten nichtaromatischen 5- bis 7-gliedrigen carbocyclischen Ring.

Umsetzungen von Verbindungen der Formel II mit den Hydrazinen der Formel III oder deren Salzen werden bevorzugt in einem Lösungsmittel oder Dispergiermittel vorgenommen. Geeignete Lösungsmittel sind zum Beispiel Wasser, Alkohole wie Methanol, Ethanol, n-Propanol, Isopropanol oder Butanole, Ether wie Diethylether, Dipropylether, Dibutylether, Methyl-tert-butylether, Tetrahydrofuran oder Dioxan, Monoether und Diether des Ethylenglykols und des Di- und Triethylenglykols wie Ethylenglykolmonomethylether, Ethylenglykolmonoethylether, Ethylenglykoldimethylether, Ethylenglykolmonobutylether, Diethylenglykolmonomethylether oder Diethylenglykoldimethylether, Ester wie Essigsäureethylester oder Essigsäurebutylester, Amide wie Dimethylformamid, Dimethylacetamid, N-Methylpyrrolidon oder Hexamethylphosphorsäuretriamid, Nitrile wie Acetonitril, Säuren wie Essigsäure, Sulfoxide und Sulfone wie Dimethylsulfoxid oder Sulfolan, Kohlenwasserstoffe und chlorierte Kohlenwasserstoffe wie Benzinfraktionen, Benzol, Toluol, Xylol, Chlorbenzol, Dichlorbenzol, Methylenchlorid oder Chloroform. Es können auch Mischungen von zwei oder mehr Lösungsmitteln eingesetzt werden, beispielsweise Mischungen aus Wasser und Alkoholen, Mischungen aus Wasser und Säuren oder Mischungen aus Alkoholen und Säuren. Bevorzugte Lösungsmittel sind Alkohole wie Methanol und Ethanol.

Die Umsetzung wird im allgemeinen bei Temperaturen von 0 °C bis 150 °C, bevorzugt bei Temperaturen von 20 °C bis 130 °C durchgeführt. Besonders bevorzugt ist, sie unter Rückfluß bei der Siedetemperatur des verwendeten Lösungsmittels oder Lösungsmittelgemisches durchzuführen, zum Beispiel bei der Siedetemperatur des Methanols oder Ethanols. Die Reaktionsdauer richtet sich nach dem Einzelfall und hängt zum Beispiel von der Reaktivität der Reaktionspartner und den Reaktionsbedingungen ab. Im allgemeinen ist, wenn die Umsetzung in Methanol oder Ethanol bei Siedetemperatur durchgeführt wird, die Umsetzung nach 1 bis 10 Stunden beendet. Die Aufarbeitung des Reaktionsgemisches kann nach Standardverfahren erfolgen und das Produkt gewünschtenfalls nach üblichen Reinigungsmethoden, zum Beispiel durch Umkristallisation oder Chromatographie, gereinigt werden.

Werden freie Hydrazine der Formel III eingesetzt, so ist es vielfach besonders vorteilhaft, die Umsetzung mit den Dicarbonylverbindungen der Formel II unter saurer Katalyse durchzuführen. Als Katalysatoren kommen zum Beispiel in Betracht organische Carbonsäuren und Sulfonsäuren wie Essigsäure, Trifluoressigsäure, Methansulfonsäure oder p-Toluolsulfonsäure, anorganische Säuren wie Chlorwasserstoff, Schwefelsäure oder Phosphorsäure, saure Salze wie Ammoniumsalze oder Hydrogenphosphate, oder saure Ionenaustauscher. Es kann auch günstig sein, einen

bestimmten pH-Wert einzustellen oder in Gegenwart eines Puffersystems zu arbeiten. Durch einen sauren Katalysator kann auch die Dicarbonylverbindung der Formel II aus einer Vorstufe, zum Beispiel einem Acetal oder Ketal, freigesetzt werden, die Dicarbonylverbindung kann also in Form einer solchen Vorstufe eingesetzt werden. Bevorzugt ist es, die Umsetzung von Verbindungen der Formel II mit freien Hydrazinen der Formel III in Gegenwart von Essigsäure durchzuführen. Die Art und Menge eines zugesetzten sauren Katalysators richten sich nach dem Einzelfall und hängen zum Beispiel von der Reaktivität der Reaktionspartner, dem Lösungsmittel oder der vorgesehenen Temperatur ab. Wird zum Beispiel eine Säure wie Essigsäure verwendet, so kann diese je nach der eingesetzten Menge sowohl als Lösungsmittel als auch als Katalysator fungieren. Wird an Stelle eines freien Hydrazins ein Säureadditionssalz eines Hydrazins eingesetzt, zum Beispiel ein $R^{4'}$-$(CH_2)_n$-substituiertes Hydraziniumchlorid oder Hydraziniumsulfat, so wird damit bereits eine saure Verbindung in das Reaktionsgemisch eingebracht, die katalytisch wirken kann. Bei Verwendung eines Hydraziniumsalzes ist es vielfach vorteilhaft, zur Einstellung eines günstigen pH-Wertes einen Teil der eingebrachten Säure durch Zusatz einer gewissen Menge einer Base abzufangen, zum Beispiel durch Zusatz von Natriumacetat oder einer anderen abstumpfenden Substanz zum Reaktionsgemisch.

Das Mengenverhältnis, in dem die Verbindungen der Formeln II und III vorteilhafterweise in die Reaktion eingesetzt werden, hängt vom Einzelfall ab. Es kann ungefähr 1:1 1 betragen, es kann aber auch ein Reaktionspartner in einem kleinerem oder in einem größerem Überschuß eingesetzt werden. Wenn zum Beispiel ein Reaktionspartner aufwendig in einer mehrstufigen Synthese hergestellt wird und der andere Reaktionspartner einfach zugänglich ist, kann es zur weitgehenden Ausnutzung des ersteren günstig sein, den letzteren im Überschuß einzusetzen, zum Beispiel in 1.1- bis 5-facher molarer Menge.

Bei der Umsetzung der Verbindungen der Formeln II und III zur Verbindung der Formel le' kann als Zwischenprodukt zunächst das Hydrazon der Formel IV entstehen, in der die Reste und n die oben für die Formeln II und III angegebenen Bedeutungen haben und das je nach den angewandten Reaktionsbedingungen isolierbar sein kann. Je nach dem Einzelfall kann es günstig sein, die Reaktion durch die Wahl der Reaktionsbedingungen wie Lösungsmittel, Temperatur und Katalysator so zu führen, daß sie zunächst nur zum Hydrazon der Formel IV führt, und dieses dann in einem separaten Schritt zum Pyrazol der Formel le' zu cyclisieren. Es kann aber auch günstig sein, die Reaktion so zu führen, daß direkt das Pyrazol entsteht. Wird die Reaktion in zwei Stufen durchgeführt, so kann für die Cyclisierung des Hydrazons der Formel IV dieses in Substanz isoliert werden oder es kann das Reaktionsgemisch der Hydrazonherstellung eingesetzt werden und die Cyclisierung zum Beispiel durch eine Erhöhung der Temperatur und/oder durch Zusatz eines Katalysators bewirkt werden.

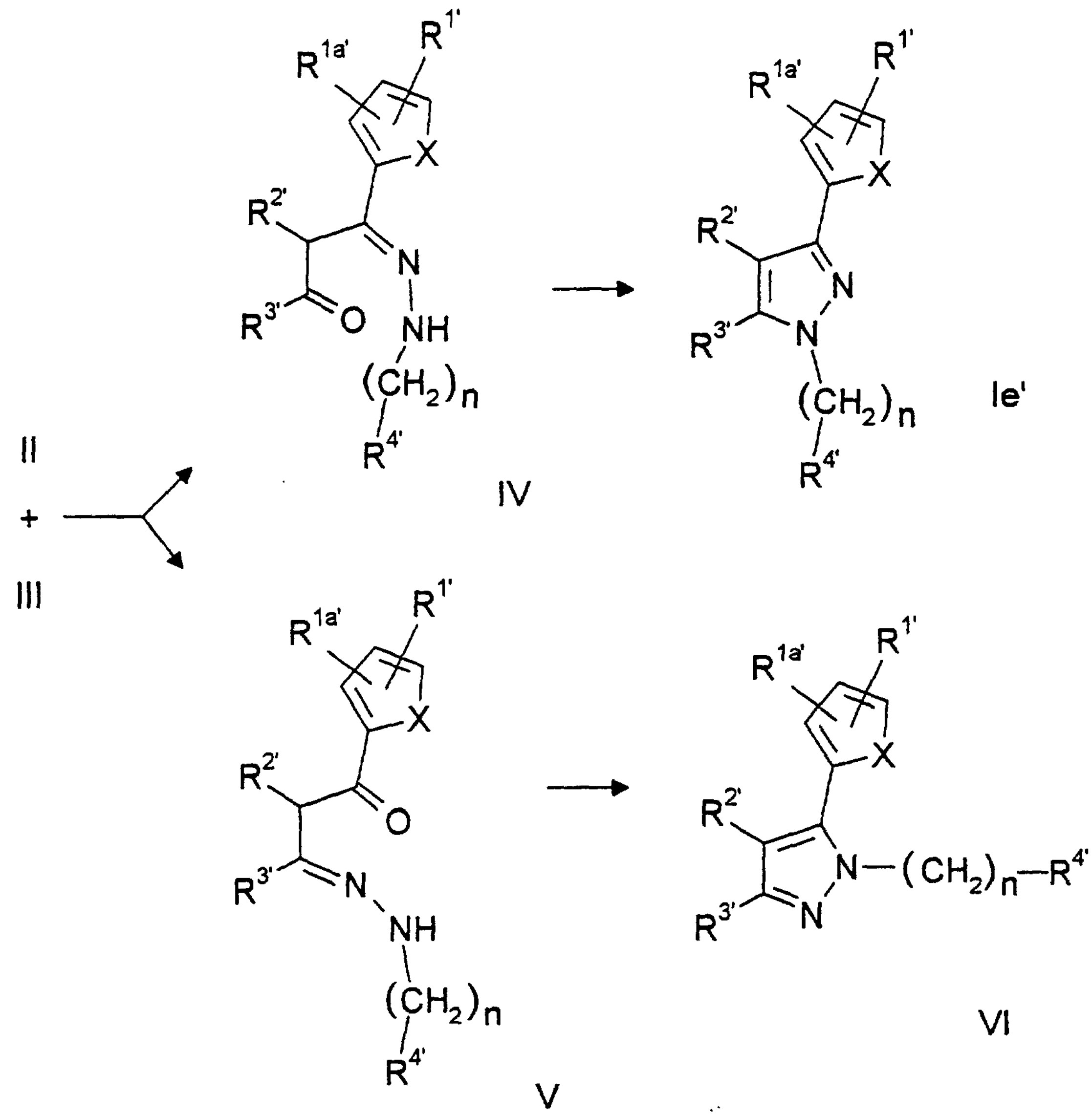

II + III → IV → Ie'

V → VI

Je nach den Reaktionsbedingungen und den Reaktivitäten der Reaktionspartner kann die $NH_2$-Gruppe des Hydrazins der Formel III statt mit derjenigen Carbonylgruppe, die dem Heterocyclus in der Formel II benachbart ist, auch mit derjenigen Carbonylgruppe reagieren, die dem Rest $R^{3'}$ benachbart ist. In diesem Falle kann die Umsetzung auch zu dem unerwünschten isomeren Pyrazol der Formel VI führen, wobei als Zwischenstufe wiederum zunächst ein Hydrazon der Formel V entstehen kann, das gegebenenfalls isoliert werden kann. In den Formeln V und VI haben die Reste und n die oben für die Formeln II und III angegebenen Bedeutungen. Liefert die Umsetzung der Verbindungen der Formeln II und III Gemische der isomeren Pyrazole Ie' und VI, so können diese nach üblichen Verfahren in die Komponenten aufgetrennt werden, zum Beispiel durch Umkristallisation oder insbesondere durch Chromatographie. Eine Auftrennung eines Isomerengemisches kann auch auf der Stufe der Hydrazone erfolgen.

Erfindungsgemäße Verbindungen der Formel I, in der die Reste $R^2$ und $R^3$ zusammen mit den sie tragenden Kohlenstoffatomen einen Benzolring bilden, also Verbindungen der Formel Ia, können dadurch dargestellt werden, daß Verbindungen der Formel VII mit Hydrazinen der Formel III oder deren Salzen umgesetzt werden. In den Formeln VII, VIII und Ia' können die Reste X, $R^{1'}$, $R^{1a'}$, $R^{4'}$, $R^{5'}$ und die Zahl n die oben angegebenen Bedeutungen von X, $R^1$, $R^{1a}$, $R^4$, $R^5$ und n haben, in diesen Resten können aber auch funktionelle Gruppen in geschützter Form bzw. in Form von Vorstufen vorliegen. y hat in den Formeln Ia', VII und VIII die oben angegebenen Bedeutungen, y steht kann hier also für 0, 1, 2, 3 oder 4 stehen. $Z^1$ in den Formeln VII und VIII steht für eine Abgangsgruppe, zum Beispiel für Halogen oder für andere geeignete Gruppen wie den Trifluormethansulfonyloxyrest. Bevorzugt steht $Z^1$ für Fluor. Aus den Verbindungen der Formel Ia' können dann gegebenenfalls erfindungsgemäße Verbindungen der Formel Ia erhalten werden, indem man in einem anschließenden Reaktionsschritt die in geschützter Form bzw. in Form von Vorstufen vorliegenden Gruppen in die gewünschten, in den obigen Definitionen von $R^1$, $R^{1a}$, $R^4$ und $R^5$ genannten funktionellen Gruppen überführt.

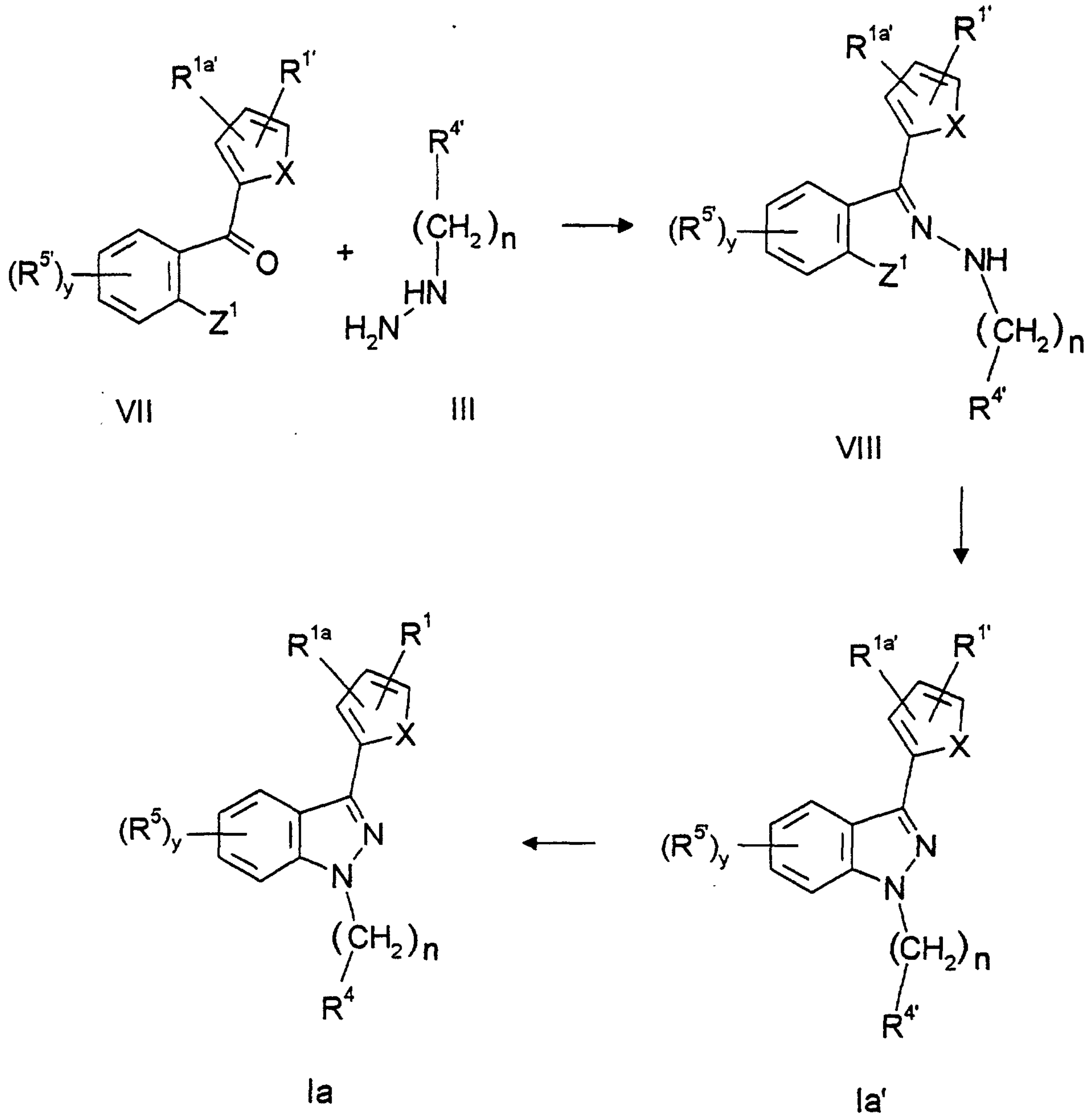

VII III VIII

Ia Ia'

Wie bei der Umsetzung von Verbindungen der Formeln II und III kann auch bei der Umsetzung von Verbindungen der Formeln VII und III als Zwischenprodukt zunächst ein Hydrazon entstehen, also eine Verbindung der Formel VIII, das je nach der Reaktivität der eingesetzten Ausgangssubstanzen und den Reaktionsbedingungen isolierbar sein kann. Je nach dem Einzelfall kann es günstig sein, die Umsetzung der Verbindungen der Formeln III und VII durch die Wahl der Reaktionsbedingungen wie Lösungsmittel, Temperatur und Katalysator so zu führen, daß sie zunächst nur zum Hydrazon der Formel VIII führt, und dieses dann in einem separaten Schritt zum Indazol der Formel Ia' zu cyclisieren, es kann aber auch günstig sein, die Reaktion so zu führen, daß direkt das Indazol entsteht. In anderen Fällen wiederum kann es aufgrund der Reaktivitäten der Ausgangsverbindungen angebracht sein, die Reaktion in zwei Stufen durchzuführen und in der zweiten Stufe, der Cyclisierung des Hydrazons der Formel VIII zum Indazol der Formel Ia', die Reaktionsbedingungen abzuändern. So ist es zum Beispiel, wenn die Gruppe $Z^1$ in der Verbindung der Formel VII bzw. der Formel VIII nicht durch Substituenten im Benzolring aktiviert ist, im allgemeinen angebracht, zunächst in Gegenwart eines sauren Katalysators die Verbindungen der Formeln VII und III zum Hydrazon der Formel VIII zu kondensieren und die Cyclisierung dann in einem zweiten Schritt vorzunehmen, in dem man in Gegenwart einer Base arbeitet, durch die die Nucleophilie des β-Stickstoffatoms in der Hydrazongruppierung erhöht wird. Wird die Reaktion

in zwei Stufen durchgeführt, so kann für die Cyclisierung des Hydrazons der Formel VIII dieses in Substanz isoliert werden oder es kann ohne Isolierung des Hydrazons das Reaktionsgemisch der Hydrazonherstellung in die Cyclisierung eingesetzt werden.

Die obigen Erläuterungen zur Umsetzung der Verbindungen der Formeln II und III hinsichtlich Lösungsmittel, Reaktionstemperaturen, Katalysatoren, Mengenverhältnissen, usw. gelten für die Umsetzung der Verbindungen der Formeln VII und III ensprechend. Auch die Umsetzungen von Verbindungen der Formel VII mit den Hydrazinen der Formel III oder deren Salzen werden bevorzugt in einem Lösungsmittel oder Dispergiermittel vorgenommen. Geeignete Lösungsmittel sind auch hier zum Beispiel Wasser, Alkohole, Ether, Monoether und Diether des Ethylenglykols und des Di- und Triethylenglykols, Ester, Amide, Nitrile, Säuren, Sulfoxide und Sulfone, Kohlenwasserstoffe und chlorierte Kohlenwasserstoffe. Beispiele für diese Lösungsmittel sind oben genannt. Es können auch Mischungen von zwei oder mehr Lösungsmitteln eingesetzt werden. Bevorzugte Lösungsmittel sind auch hier Alkohole wie Methanol und Ethanol. Auch hier wird die Umsetzung im allgemeinen bei Temperaturen von 0 °C bis 150 °C, bevorzugt bei Temperaturen von 20 °C bis 130 °C durchgeführt. Besonders bevorzugt ist, sie unter Rückfluß bei der Siedetemperatur des verwendeten Lösungsmittels durchzuführen, zum Beispiel bei der Siedetemperatur des Methanols oder Ethanols.

Werden freie Hydrazine der Formel III eingesetzt, so ist es vielfach besonders vorteilhaft, die Umsetzung mit den Verbindungen der Formel VII unter saurer Katalyse durchzuführen. Die obigen Erläuterungen gelten wiederum auch hier. Auch hier ist es bevorzugt, die Umsetzung von Verbindungen der Formeln VII mit freien Hydrazinen der Formel III in Gegenwart von Essigsäure durchzuführen. Wird an Stelle eines freien Hydrazins ein Säureadditionssalz eines Hydrazins eingesetzt, ist es vielfach wiederum vorteilhaft, zur Einstellung eines günstigen pH-Wertes einen Teil der eingebrachten Säure durch Zusatz einer gewissen Menge einer Base abzufangen, zum Beispiel durch Zusatz von Natriumacetat.

Wie bereits erwähnt, kann die Umsetzung der Verbindungen der Formeln VII und III auf der Stufe des Hydrazons der Formel VIII unterbrochen werden. Die dann in einem zweiten Schritt erfolgende Cyclisierung des Hydrazons zum Indazol der Formel Ia', die eine nucleophile Substitution der Gruppe $Z^1$ am Aromaten durch das β-Stickstoffatom der Hydrazonogruppe darstellt, kann je nach den im Einzelfall gegebenen Reaktivitäten zum Beispiel durch Erhitzen in einem Lösungsmittel oder Dispergiermittel erfolgen. In vielen Fällen ist es vorteilhaft, dabei eine Base zuzusetzen. Wenn bei der Herstellung von Verbindungen der Formel Ia' die Cyclisierung in einem separaten Schritt erfolgt, ist es bevorzugt, in diesem Schritt in Gegenwart einer Base zu arbeiten. Als Basen kommen zum Beispiel Hydroxide, Carbonate, Hydrogencarbonate, Hydride, Amide, Alkoholate oder metallorganische Verbindungen von Alkalimetallen wie Lithium, Natrium, Kalium oder Cesium oder Erdalkalimetallen wie Magnesium oder Calcium in Betracht. Bevorzugte Basen sind Alkalimetallalkoholate von ($C_1$-$C_4$)-Alkanolen wie Natrium- und Kaliummethylat, Natrium- und Kaliumethylat oder Natrium- und Kalium-tert-butylat. Es können auch Gemische von zwei oder mehr Basen eingesetzt werden. Die Base wird bevorzugt in äquimolarer Menge oder im Überschuß eingesetzt, üblicherweise in 1- bis 3-facher molarer Menge.

Als Lösungsmittel oder Dispergiermittel für eine in einem separaten Schritt durchgeführte Cyclisierung eines Hydrazons der Formel VIII zum Indazol kommen zum Beispiel Wasser, Alkohole, Ether, Monoether und Diether des Ethylenglykols und des Di- und Triethylenglykols, Ester, Amide, Nitrile, Sulfoxide, Sulfone, Kohlenwasserstoffe und chlorierte Kohlenwasserstoffe in Betracht. Beispiele für diese Lösungsmittel sind oben genannt. Es können auch Mischungen von zwei oder mehr Lösungsmitteln eingesetzt werden. Bevorzugte Lösungsmittel für eine Cyclisierung in Gegenwart einer Base sind aprotische Lösungsmittel, insbesondere dipolar aprotische Lösungsmittel wie Dimethylformamid, Dimethylacetamid, Hexamethylphosphorsäuretriamid, N-Methylpyrrolidon oder Dimethylsulfoxid.

Eine in einem separaten Schritt erfolgende Cyclisierung des Hydrazons der Formel VIII wird im allgemeinen bei Temperaturen von 0 °C bis 150 °C, bevorzugt bei Temperaturen von 20 °C bis 130 °C durchgeführt. Besonders bevorzugt ist es wiederum, sie unter Rückfluß bei der Siedetemperatur des verwendeten Lösungsmittels oder Lösungsmittelgemisches durchzuführen. Die Aufarbeitung kann nach Standardmethoden erfolgen.

Ein weiteres Verfahren zur Herstellung von Verbindungen der Formel Ia bzw. Ia' neben der Umsetzung von Verbindungen der Formel VII mit $R^{4'}$-$(CH_2)_n$-substituierten Hydrazinen der Formel III ist die Umsetzung von Verbindungen der Formel VII mit Acylhydrazinen der Formel IX. In der Formel IX kann R beispielsweise für einen Alkylrest stehen, zum Beispiel für einen ($C_1$-$C_4$)-Alkylrest wie den Methylrest oder den tert-Butylrest, oder für einen Arylrest, zum Beispiel einen Phenylrest, der unsubstituiert oder substituiert sein kann. Ein Beispiel für eine geeignete Verbindung der Formel IX ist das Benzhydrazid. Die Umsetzung der Verbindungen der Formeln VII und IX kann unter den gleichen Bedingungen erfolgen wie oben für Umsetzung der Verbindungen der Formeln VII und III angegeben. Alle obigen Erläuterungen gelten hier entsprechend. Die Umsetzung der Verbindungen der Formeln VII und IX, die wiederum in einem Schritt oder in zwei Schritten erfolgen kann, führt zunächst zu den den Formeln VIII bzw. Ia' entsprechenden Acylhydrazonen bzw. Acylindazolen, die an Stelle der Gruppe $R^{4'}$-$(CH_2)_n$ die Gruppe R-CO enthalten. Nach Abspaltung der Acylgruppe werden aus diesen Verbindungen die Indazole der Formel X erhalten, in der X, $R^{1'}$, $R^{1a'}$, $R^{5'}$

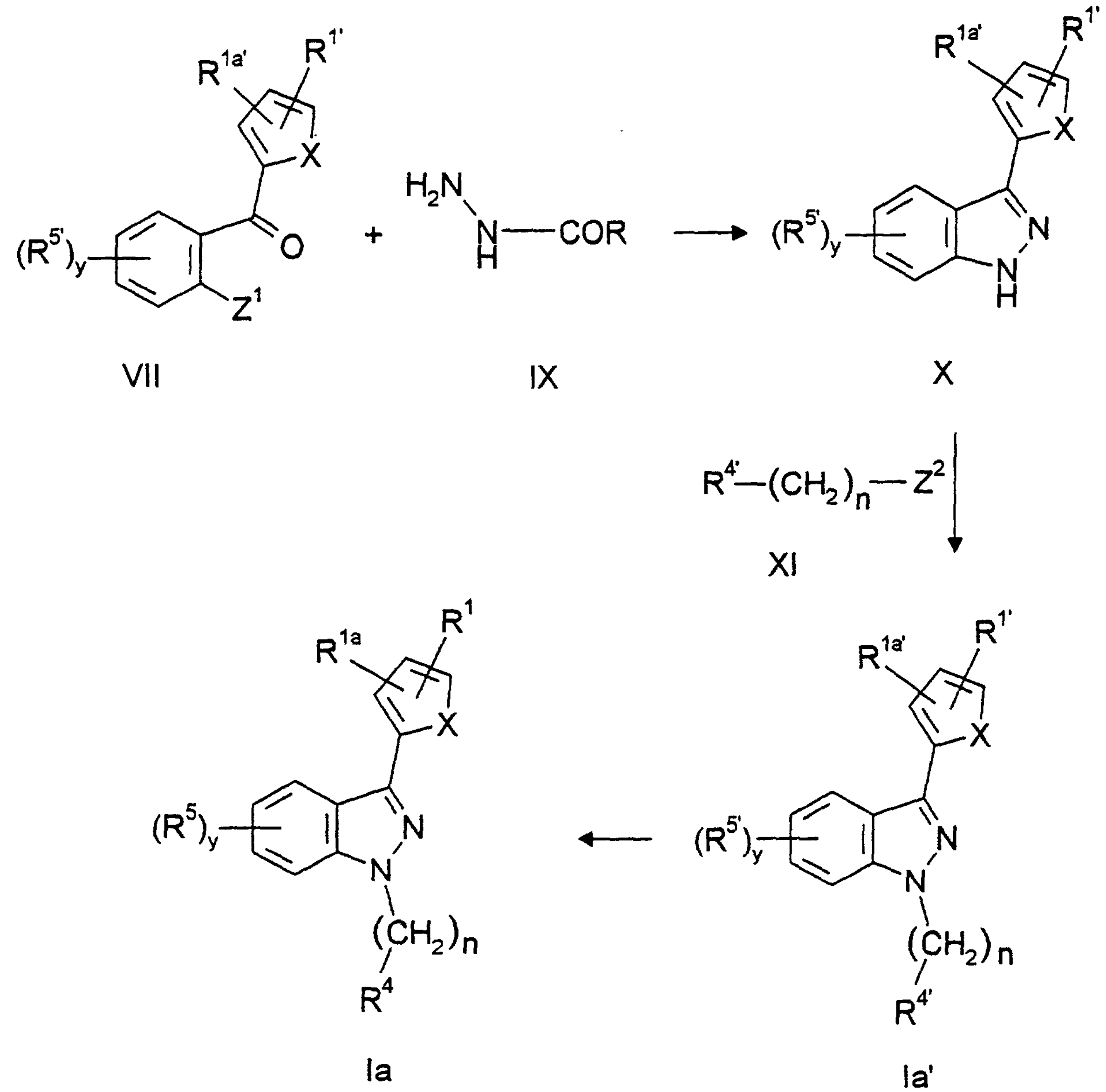

und y die oben für die Formeln Ia', VII und VIII angegebenen Bedeutungen haben. Für die Abspaltung der Acylgruppe können die Acylverbindungen zunächst isoliert werden, die Abspaltung kann aber auch ohne deren Isolierung in situ erfolgen. Die Abspaltung der Acylgruppe läßt sich zum Beispiel in üblicher Weise durch Hydrolyse unter sauren oder basischen Bedingungen durchführen, zum Beispiel mittels Salzsäure, Schwefelsäure, Phosphorsäure, Lithiumhydroxid, Natriumhydroxid, Natriumcarbonat oder Kaliumhydroxid. Sie kann in Wasser oder in einem wasserhaltigen organischen Lösungsmittel oder Dispergiermittel erfolgen. Die Reaktionstemperatur und Reaktionsdauer richten sich nach dem Einzelfall, im allgemeinen wird bei Raumtemperatur bis 100 °C gearbeitet. Die in der 1-Position unsubstituierten Indazole der Formel X können nach den üblichen Methoden isoliert werden oder auch direkt in eine Folgereaktion eingesetzt werden. Die Indazole der Formel X können dann durch Umsetzung mit Alkylierungsmitteln der Formel XI in die 1-substituierten Indazole der Formel Ia' überführt werden. In der Formel XI haben $R^{4'}$ und n die oben angegebenen Bedeutungen, $Z^2$ steht für eine Abgangsgruppe, beispielsweise für Chlor, Brom, Iod oder einen Sulfonyloxyrest wie Methansulfonyloxy, Trifluormethansulfonyloxy, Benzolsulfonyloxy oder p-Toluolsulfonyloxy. Im allgemeinen wird das Alkylierungsmittel der Formel XI in äquimolarer Menge oder im Überschuß eingesetzt, zum Beispiel in 1- bis 3-facher molarer Menge.

Die Alkylierung der Verbindungen der Formel X kann unter den üblichen Alkylierungsbedingungen erfolgen. Bevorzugt wird sie in einem Lösungsmittel oder Dispergiermittel durchgeführt, zum Beispiel in Wasser, einem Alkohol, einem Ether, einem Monoether oder Diether des Ethylenglykols oder des Di- und Triethylenglykols, einem Keton wie Aceton oder Methylethylketon, einem Ester, einem Amid, einem Nitril, einem Sulfoxid oder Sulfon, einem Kohlenwasserstoff oder einem chlorierten Kohlenwasserstoff. Die oben angegebenen Beispiele für diese Lösungsmittel gelten auch hier. Es können auch Mischungen von zwei oder mehr Lösungsmitteln eingesetzt werden, beispielsweise Mischungen eines organischen Lösungsmittels mit Wasser. Bevorzugt wird die Alkylierung in Wasser oder in einem dipolar aprotischen Lösungsmittel, zum Beispiel Dimethylformamid, durchgeführt. Die Alkylierung wird im allgemeinen bei Temperaturen von 0 °C bis 150 °C, bevorzugt bei Temperaturen von 20 °C bis 130 °C durchgeführt. Besonders bevorzugt ist, sie unter Rückfluß bei der Siedetemperatur des verwendeten Lösungsmittels durchzuführen.

Die Alkylierung der Verbindungen der Formel X mit den Verbindungen der Formel XI erfolgt bevorzugt unter Zusatz einer Base. Geeignete Basen sind zum Beispiel die Hydroxide, Carbonate, Acetate, Hydride oder Alkoholate von Alkalimetallen wie Lithium, Natrium oder Kalium oder von Erdalkalimetallen wie Magnesium oder Calcium. Es können auch Gemische von Basen verwendet werden. Im allgemeinen wird die Base in äquimolarer Menge oder im Überschuß eingesetzt, zum Beispiel in 1- bis 3-facher molarer Menge. Besonders bevorzugt sind die Alkylierung unter Verwendung von Kalium-tert-butylat oder Natriumhydrid in Dimethylformamid und die Verwendung von Natriumhydroxid in Wasser. Die Aufarbeitung des Reaktionsgemisches kann nach Standardverfahren erfolgen und das Produkt gewünschtenfalls nach üblichen Reinigungsmethoden, zum Beispiel durch Umkristallisation oder Chromatographie, gereinigt werden.

In den Verbindungen der Formeln Ia' und Ie' können, wie schon oben gesagt, die Reste $R^{1'}$, $R^{1a'}$, $R^{2'}$, $R^{3'}$, $R^{4'}$ und $R^{5'}$ sowie y die in den Definition von $R^1$, $R^{1a}$, $R^2$, $R^3$, $R^4$ und $R^5$ angegebenen Bedeutungen haben, so daß die nach den erläuterten Syntheseverfahren erhaltenen Reaktionsprodukte der Formeln Ia' und Ie' bereits erfindungsgemäße Verbindungen der Formel Ia und Ie darstellen. Es können aber in den nach den erläuterten Syntheseverfahren erhaltenen Verbindungen der Formeln Ia' und Ie' auch noch in vielfältiger Weise strukturelle Abwandlungen vorgenommen werden. Wie schon gesagt, kann es sich dabei um die Freisetzung von funktionellen Gruppen handeln, die während der Synthese in geschützter Form vorlagen. Es können aber auch in erfindungsgemäße Verbindungen der Formeln Ia' und Ie' nach üblichen chemischen Methoden zusätzliche funktionelle Gruppen eingeführt werden oder in erfindungsgemäßen Verbindungen vorhandene Strukturelemente oder funktionelle Gruppe nach üblichen Methoden abgewandelt werden. Diese Methoden sind dem Fachmann bekannt und in Standardwerken ausführlich beschrieben, zum Beispiel in Houben-Weyl, Methoden der Organischen Chemie, Thieme-Verlag, Stuttgart, oder Organic Reactions, John Wiley & Sons, New York. Eine gegebenenfalls notwendige Anpassung von Reaktionsbedingungen an die Reaktivität der Verbindungen der Formeln Ia' und Ie' bereitet dem Fachmann keine Probleme. Beispielhaft seien als in Betracht kommende Reaktionstypen genannt:

Hydrolyse von Carbonsäureestern zu den Carbonsäuren bzw. den Alkoholen

Überführung von Carbonsäuren in die Carbonsäureester durch Veresterung mit Alkoholen in Gegenwart von Säure, Überführung von Carbonsäuren in Carbonsäureamide (= Aminocarbonylverbindungen oder Carbamoylverbindungen) oder in Carbonsäureester durch in situ-Aktivierung der Carbonsäuren und Umsetzung mit Aminen oder Alkoholen oder Überführung von Carbonsäuren in reaktive Derivate, zum Beispiel in die Carbonsäurechloride durch Umsetzung der Säuren oder ihrer Salze mit Chlorierungsmitteln wie Thionylchlorid, Oxalylchlorid oder Phosphorhalogeniden, und Umsetzung der reaktiven Derivate mit Alkoholen und Aminen zu Carbonsäureestern und Carbonsäureamiden

Überführung von Sulfonsäuren oder ihren Salzen in die Sulfonsäurechloride zum Beispiel mit Phosphorhalogeniden, und Umsetzung der Sulfonsäurechloride mit Aminen zu Sulfonsäureamiden

Reduktion von Carbonsäurederivaten zu Aldehyden oder Alkoholen und Reduktion von Aldehyden und Ketonen zu Alkoholen sowie Addition von metallorganischen Verbindungen wie Grignard-Verbindungen oder lithiumorganischen Verbindungen an Carbonsäuregruppen oder Aldehyd- oder Ketongruppen unter Bildung von Ketonen oder Alkoholen

Oxidation von Alkoholen zu Aldehyden oder Ketonen

Veretherung, Halogenierung und Veresterung von Alkoholen

Reduktion von Nitroverbindungen zu Aminen

Acylierung von Aminen zu Carbonsäureamiden mit Carbonsäuren in Gegenwart eines aktivierenden Agens oder mit reaktiven Carbonsäurederivaten wie Carbonsäurechloriden, sowie Umsetzung von Aminen mit Sulfonsäurechloriden zu Sulfonsäureamiden

Nucleophile Substitutionen an aliphatischen Kohlenstoffatomen, zum Beispiel Umsetzung von Halogenverbindungen mit Aminen oder Mercaptanen

Elektrophile aromatische Substitution unter Ersatz eines Wasserstoffatoms an einem carbocyclischen oder heterocylischen aromatischen Ring durch eine funktionelle Gruppe, zum Beispiel Halogenierung, Aminomethylierung, Formylierung, Acylierung, Sulfonierung.

Die Ausgangsprodukte der Formeln II und VII sind bekannt oder können analog zu bekannten Verbindungen nach wohlbekannten, in der Literatur beschriebenen Standardverfahren hergestellt werden. 1,3-Dicarbonylverbindungen der Formel II sind zum Beispiel durch Esterkondensationen oder durch Acylierungen von β-Ketoestern mit anschließender Abspaltung der Estergruppe erhältlich. Die Aromaten der Formel VII können zum Beispiel durch Friedel-Crafts-Acylierungen von Furanen, Thiophenen und Pyrrolen mit Benzoesäurederivaten wie zum Beispiel Säurechloriden erhalten werden. Näheres zu diesen Reaktionen findet sich in den Standardwerken wie Houben-Weyl oder Organic Reactions (siehe oben). Auch die Hydrazine der Formeln III und IX sowie die Alkylierungsmittel der Formel XI sind bekannt oder können nach wohlbekannten Verfahren hergestellt werden, zu denen sich in in diesen Standardwerken nähere Angaben finden.

Die erfindungsgemäßen Verbindungen der Formel 1 bewirken über die Aktivierung der löslichen Guanylat-Cyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung des vorhandenen cGMP-Spiegels angestrebt wird. Die Aktivierung der sGC durch die Verbindungen der Formel I kann zum Beispiel in dem unten beschriebenen Aktivitätsassay untersucht werden, ihre Organwirkung zum Beispiel durch die Bestimmung der Relaxation der Ratten-Aorta.

Krankheiten und pathologische Zustände, die mit einem niedrigen cGMP-Spiegel verbunden sind oder bei denen eine Erhöhung des cGMP-Spiegels angestrebt wird und zu deren Therapie und Prophylaxe Verbindungen der Formel I eingesetzt werden können, sind zum Beispiel Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei der Therapie der Leberzirrhose sowie aufgrund ihrer zum Teil synergistischen Wirkung mit der retrograden Messenger-Substanz NO zur Verbesserung einer eingeschränkten Lernfähigkeit oder Gedächtnisleistung.

Die Verbindungen der Formel I und ihre physiologisch verträglichen Salze können somit am Tier, bevorzugt am Säugetier; und insbesondere am Menschen als Arzneimittel für sich allein, in Mischungen untereinander oder in Form von pharmazeutischen Zubereitungen verwendet werden. Gegenstand der vorliegenden Erfindung sind daher auch die Verbindungen der Formel I und ihre physiologisch verträglichen Salze zur Verwendung als Arzneimittel, ihre Verwendung zur Normalisierung eines gestörten cGMP-Haushalts und insbesondere ihre Verwendung in der Therapie und Prophylaxe der oben genannten Krankheitsbilder, sowie ihre Verwendung zur Herstellung von Medikamenten dafür. Weiterhin sind Gegenstand der vorliegenden Erfindung pharmazeutische Präparate, die als aktiven Bestandteil eine wirksame Dosis mindestens einer Verbindung der Formel I und/oder eines physiologisch verträglichen Salzes davon neben üblichen pharmazeutisch einwandfreien Trägerstoffen und Zusatzstoffen enthalten.

Die Arzneimittel können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die pharmazeutischen Präparate enthalten normalerweise 0.5 bis 90 Gewichtsprozent der Verbindungen der Formel I und/oder ihrer physiologisch verträglichen Salze. Die Herstellung der pharmazeutischen Präparate kann in an sich bekannter Weise erfolgen. Dazu werden ein oder mehrere Verbindungen der Formel I und/oder ihre physiologisch verträglichen Salze zusammen mit einem oder mehreren festen oder flüssigen galenischen Trägerstoffen und/ oder Hilfsstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann.

Für die Herstellung beispielsweise von Pillen, Tabletten, Dragees und Hartgelatinekapseln kann man Lactose,

Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze können auch lyophilisiert werden und die erhaltenen Lyophilisate zum Beispiel zur Herstellung von Injektions- oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel oder Antioxidantien.

Die Dosierung des zu verabreichenden Wirkstoffs der Formel I und/oder eines physiologisch verträglichen Salze davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden. Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung wirksamer Ergebnisse angemessen. Die Tagesdosis kann in einer Einzeldosis verabreicht werden oder, insbesondere bei der Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0.2 bis 500 mg, vorzugsweise 1 bis 200 mg Wirkstoff der Formel I und/oder dessen physiologisch verträgliche Salze pro Dosis.

Die Verbindungen der Formel I aktivieren die lösliche Guanylatcyclase. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zell- oder Gewebsproben. Ferner können die Verbindungen der Formel I und ihre Salze, wie bereits oben erwähnt, als Zwischenprodukte zur Herstellung weiterer Arzneimittelwirkstoffe dienen.

Die nachfolgenden Beispiele erläutern die Erfindung, ohne sie einzuschränken.

In den Versuchsbeschreibungen bedeuten:

RT Raumtemperatur
THF Tetrahydrofuran
DMF Dimethylformamid

Beispiel 1

1-Benzyl-3-(5-carboxy-2-furyl)-indazol-Kaliumsalz (Ausgangsmaterial)

1 a) 5-(2-Fluor-benzoyl)-furan-2-carbonsäure

132.6 g (0.53 mol) 5-(2-Fluor-benzoyl)-furan-2-carbonsäuremethylester wurden in eine Lösung aus 31.47 g (0.56 mol) Kaliumhydroxid in 350 ml Wasser eingetragen und auf 70 °C erwärmt. Nach 1 h wurde die Lösung filtriert und mit konz. Salzsäure sauer gestellt. Der Niederschlag wurde abgesaugt und aus Ethanol umkristallisiert. Man erhielt 106.3 g (85 %) der Titelverbindung.
Schmp.: 195-196 °C

1 b) 1-Benzyl-3-(5-carboxy-2-furyl)-indazol-Kaliumsalz

70.26 g (0.3 mol) 5-(2-Fluor-benzoyl)-furan-2-carbonsäure wurden in 350 ml Methanol vorgelegt, 109.95 g (0.9 mol) Benzylhydrazin wurden zugesetzt und nach Zugabe von 8 ml Eisessig wurde 6 h unter Rückfluß erhitzt. Zur Aufarbeitung wurde einrotiert, der Rückstand in 2 N Natronlauge aufgenommen und mit Essigester extrahiert. Die wäßrige Phase wurde mit 2 N Salzsäure sauer gestellt und mit Essigester extrahiert. Der nach Trocknen und Einrotieren verbliebene Rückstand wurde aus Essigester/n-Hexan umkristallisiert. Die auf diese Weise erhaltenen 68.3 g (0.20

mol) 5-(2-Fluor-benzoyl)-furan-2-carbonsäure-Benzylhydrazon (Schmp.: 147 °C (Zers.)) wurden in 400 ml DMF gelöst, 45.38 g (0.40 mol) Kalium-tert-butylat wurden eingetragen und es wurde 30 min unter Rückfluß erhitzt. Der ausgefallene Niederschlag wurde abgesaugt, mit Dichlormethan gewaschen und aus Ethanol/Wasser (95:5) umkristallisiert. Man erhielt 57.7 g (54 %) der Titelverbindung. Die entsprechende Cyclisierung der in der Mutterlauge noch enthaltenen Vorstufe ergab 25.9 g Produkt.
Schmp.: > 300 °C
$^1$H-NMR ($D_6$-DMSO): $\delta$ = 5.73 (s, 2H, $CH_2$), 6.68 (d, 1 H, H-3'), 6.92 (d, 1 H, H-4'), 7.18-7.38 (m, 6H, Phenyl-H, H-5), 7.45 (t, 1 H, H-6), 7.75 (d, 1 H, H-7), 8.18 (d, 1 H, H-4)

Beispiel 2

1-Benzyl-3-(5-ethoxycarbonyl-2-furyl)-indazol (Ausgangsmaterial)

52.4 g (0.15 mol) 1-Benzyl-3-(5-carboxy-2-furyl)-indazol-Kaliumsalz wurden in 1250 ml Toluol vorgelegt und nach Zugabe von 200 ml abs. Ethanol und 50 ml konz. Schwefelsäure am Wasserabscheider unter Rückfluß gerührt. Nach 3 h wurde einrotiert, das verbliebene Öl in Wasser/Essigester aufgenommen und die Wasserphase abgetrennt. Die organische Phase wurde mit Wasser und 7.5 %iger $NaHCO_3$-Lösung gewaschen, mit Natriumsulfat getrocknet und eingeengt. Der Rückstand wurde aus Isopropanol umkristallisiert. Man erhielt 37.8 g (73%) der Titelverbindung.
Schmp.: 98-99 °C

Entsprechend wurden hergestellt:

Beispiel 4

3-(5-Carboxy-2-furyl)-1-(3,5-bis(trifluormethyl)-phenyl)-indazol (Ausgangsmaterial) Schmp.: 256-257 °C

Beispiel 5

3-(5-Ethoxycarbonyl-2-furyl)-1-(3,5-bis(trifluormethyl)-phenyl)-indazol (Ausgangsmaterial) Schmp.: 128-129 °C

Beispiel 8

1-Benzyl-3-(5-methoxycarbonyl-2-furyl)-5-nitro-indazol (Ausgangsmaterial)

8a) 5-(2-Fluor-5-nitro-benzoyl)-furan-2-carbonsäuremethylester

Zu einer Suspension von 2 g Eisen-(III)-chlorid und 29.0 g (0.14 mol) 2-Fluor-5-nitro-benzoylchlorid in 100 ml getrocknetem Tetrachlorkohlenstoff wurde eine Lösung von 24.7 g (0.2 mol) Furan-2-carbonsäuremethylester in 50 ml getrocknetem Tetrachlorkohlenstoff zugetropft und 14 h unter Rückfluß (80 °C) erhitzt.

Anschließend wurden 50 ml Methanol zugesetzt, es wurde 30 min bei RT gerührt und einrotiert. Der verbliebene Rückstand wurde in Essigester aufgenommen, mit Wasser und $NaHCO_3$-Lösung gewaschen, mit Natriumsulfat getrocknet, einrotiert und aus Isopropanol umkristallisiert. Man erhielt 3.5 g (9 %) der Titelverbindung. Schmp.: 134-135 °C

8b) 1-Benzyl-3-(5-methoxycarbonyl-2-furyl)-5-nitro-indazol

2.6 g (9 mmol) 5-(2-Fluor-5-nitro-benzoyl)-furan-2-carbonsäuremethylester und 3.31 g (27 mmol) Benzylhydrazin wurden in ca. 60 ml Methanol vorgelegt und nach Zugabe von 0.2 ml Eisessig 15 min zum Rückfluß erwärmt. Der ausgefallene Niederschlag wurde abgesaugt, mit wenig Methanol gewaschen und im Vakuumtrockenschrank bei RT getrocknet. Das Rohprodukt wurde durch Chromatographie an Kieselgel mit Dichlormethan gereinigt. Man erhielt 2.9 g (85 %) der Titelverbindung.
Schmp.: 171-173 °C

Beispiel 9

5-Amino-1-benzyl-3-(5-methoxycarbonyl-2-furyl)-indazol (Ausgangsmaterial)

0.9 g (2.4 mmol) 1-Benzyl-3-(5-methoxycarbonyl-2-furyl)-5-nitro-indazol wurden durch leichtes Erwärmen in 100 ml Methanol/THF (1:1) gelöst, eine Lösung von 2.5 g (14.4 mmol) Natriumdithionit in 50 ml Wasser wurde zugegeben und das Gemisch wurde 16 h bei RT gerührt. Zur Aufarbeitung wurde eingeengt und der Rückstand an Kieselgel mit Dichlormethan/Methanol (98.5:1.5) chromatographiert. Man erhielt 140 mg (17 %) der Titelverbindung.
Schmp.: 195-196 °C

Beispiel 11

1-Benzyl-3-(5-carboxy-2-furyl)-6-nitro-indazol (Ausgangsmaterial)

11a) 5-(2-Fluor-4-nitro-benzoyl)-furan-2-carbonsäuremethylester

8.4 g Eisen-(III)-chlorid, 44.8 g (0.22 mol) 2-Fluor-4-nitro-benzoylchlorid und 33.4 g (0.26 mol) Furan-2-carbonsäuremethylester wurden in 80 ml Tetrachlorkohlenstoff 2 Tage unter Rückfluß erhitzt. Zur Aufarbeitung wurden 100 ml Methanol zugefügt, es wurde 10 min gerührt, eingeengt, der Rückstand in Essigester/Wasser aufgenommen und die organische Phase mehrfach mit $Na_2CO_3$-Lösung ausgeschüttelt. Der nach dem Trocknen und Einengen der Essigester-Phase verbliebene Rückstand wurde mit Methanol ausgezogen und der unlösliche Anteil an Kieselgel mit Dichlormethan chromatographiert. Man erhielt 19.0 g (29 %) der Titelverbindung.
Schmp.: 136-138 °C

11b) 5-(2-Fluor-4-nitro-benzoyl)-furan-2-carbonsäure

14.5 g (49 mmol) 5-(2-Fluor-4-nitro-benzoyl)-furan-2-carbonsäuremethylester wurden zu 200 ml 0.1 N Natronlauge gegeben und 3 Tage bei RT gerührt. Anschließend wurde mit 1 N Salzsäure auf pH 4 gestellt, 30 min im Eisbad gekühlt und abgesaugt. Man erhielt 8.6 g (62 %) der Titelverbindung.
Schmp.: 170 °C (Zers.)
$^1$H-NMR ($D_6$-DMSO): δ = 7.39 (d, 1H, H-4'), 7.50 (d, 1H, H-3'), 8.00 (dd, 1H, H-6), 8.25 (dd, 1H, H-5), 8.34 (dd, 1H, H-3)

11c) 1-Benzyl-3-(5-carboxy-2-furyl)-6-nitro-indazol

8.5 g (30 mmol) 5-(2-Fluor-4-nitro-benzoyl)-furan-2-carbonsäure wurden in 100 ml Methanol gelöst, mit 11.2 g (91 mmol) Benzylhydrazin versetzt und 7 h unter Rückfluß erhitzt. Anschließend wurde in Wasser gegossen, mit konz. Salzsäure auf pH 4 gestellt und mit Essigester extrahiert. Die vereinigten organischen Phasen wurden getrocknet und eingeengt. Das rohe Zwischenprodukt wurde zur Cyclisierung in 50 ml DMF gelöst, 6.8 g (61 mmol) Kalium-tert-butylat wurden zugegeben und es wurde 3 h unter Rückfluß erhitzt. Zur Aufarbeitung wurde eingeengt, mit 1 N Salzsäure auf pH 4 gestellt, mit Essigester extrahiert, die organische Phase eingeengt und der Rückstand an Kieselgel mit Essigester/Eisessig (60:1) chromatographiert. Man erhielt 8 g (ca. 73 %) der Titelverbindung als Öl.
$^1$H-NMR ($D_6$-DMSO): δ = 5.96 (s, 2H, $CH_2$), 7.22-7:41 (m, 7H, Phenyl-H, H-3', H-4'), 8.13 (dd, 1H, H-5), 8.38 (d, 1H, H-4), 8.93 (d, 1H, H-7)

Beispiel 12

3-(5-Carboxy-2-furyl)-1-(2-phenylethyl)-indazol, (Ausgangsmaterial)

5 g (21 mmol) 5-(2-Fluorbenzoyl)-furan-2-carbonsäure, 12 g (51 mmol) (2-Phenylethyl)-hydrazinium-Sulfat und 8.4 g (102 mmol) Natriumacetat wurden in 50 ml Ethanol 12 h unter Rückfluß gekocht. Anschließend wurde eingeengt, mit Wasser/Essigester verrührt, die Essigesterphase abgetrennt, getrocknet und eingeengt. Das rohe Zwischenprodukt (Hydrazon) wurde zur Reinigung an Kieselgel mit Dichlormethan/Methanol (9:1) chromatographiert. 4 g (11.3 mmol) des Hydrazons wurden in 20 ml DMF gelöst und mit 2.5 g (23 mmol) Kalium-tert-butylat 3 h unter Rückfluß erhitzt. Das ausgefallene Kaliumsalz der Titelsäure wurde abgesaugt. Das eingeengte Filtrat wurde an Kieselgel mit Dichlormethan/Methanol (7:3) chromatographiert. Man erhielt insgesamt 1.9 g (27 %, berechnet für die Säure) der Titelverbindung. Als Nebenprodukt bildete sich 3-(5-Carboxy-2-furyl)-indazol.
Schmp.: Zers. > 190 °C
$^1$H-NMR ($D_6$-DMSO): δ = 3.20 (t, 2H, $C\underline{H}_2$-Phenyl), 4.65 (t, 2H, $CH_2$-N), 7.00 (m, 2H, H-3', H-4'), 7.13-7.23 (m, 6H, Phenyl-H, H-5), 7.38 (t, 1H, H-6), 7.60 (d, 1H, H-7), 8.14 (d, 1H, H-4)

Beispiel 13

3-(5-Ethoxycarbonyl-2-furyl)-1-(2-phenylethyl)-indazol (Ausgangsmaterial)

0.8 g (2.4 mmol) 3-(5-Carboxy-2-furyl)-1-(2-phenylethyl)-indazol wurden mit 25 ml Ethanol, 150 ml Toluol und 2 ml konz. Schwefelsäure versetzt und 3 h am Wasserabscheider erhitzt. Chromatographie des nach Einrotieren erhaltenen Rückstandes an Kieselgel mit Dichlormethan/Hexan (2:1) ergab 400 mg (46 %) der Titelverbindung.
$^1$H-NMR ($D_6$-DMSO): $\delta$ = 1.40 (t, 3H, $CH_3$), 3.34 (t, 2H, $C\underline{H_2}$-Phenyl), 4.35 (q, 2H, $OCH_2$), 4.73 (t, 2H, $CH_2$-N), 7.08-7.37 (m, 6H, Phenyl-H, H-3'), 7.38 (t, 1H, H-5), 7.45 (t, 1H, H-6), 7.48 (d, 1H, H-4'), 7.65 (d, 1H, H-7), 8.10 (d, 1H, H-4)

Beispiel 15

3-(5-Carboxy-2-furyl)-indazol (Ausgangsmaterial)

2.5 g (11 mmol) 5-(2-Fluorbenzoyl)-furan-2-carbonsäure, 3.28 g (24 mmol) Benzhydrazid und 2 Tropfen Eisessig wurden in 50 ml Ethanol 10 h unter Rückfluß erhitzt. Anschließend wurde einrotiert, der Rückstand mit Wasser aufgenommen, mit 2 N $NaHCO_3$-Lösung basisch gestellt und mit Essigester extrahiert. Die wäßrige Phase wurde mit 2 N Salzsäure sauer gestellt und mit Essigester extrahiert. Die vereinigten organischen Phasen wurden getrocknet und einrotiert. Der Rückstand wurde in 25 ml DMF gelöst, 2.74 g (24.2 mmol) Kalium-tert-butylat wurden zugesetzt und es wurde 1.5 h bei Rückflußtemperatur gerührt. Nach Abkühlen wurde einrotiert, der Rückstand in 1 N Natronlauge aufgenommen und mit Essigester extrahiert. Der beim Ansäuern der wäßrigen Phase ausgefallene Niederschlag wurde abgesaugt, mit Wasser gewaschen und im Vakuumtrockenschrank bei 40 °C gefrocknet. Man erhielt 1.88 g (75 %) der Titefverbindung.
Schmp.: 205 °C (Zers.)
$^1$H-NMR ($D_6$-DMSO): $\delta$ = 7.15 (d, 1H, H-3'), 7,28 (t, 1H, H-5), 7.40 (d, 1H, H-4'), 7.48 (t, 1H, H-6), 7.63 (d, 1 H, H-7), 8.15 (d, 1H, H-4), 13.1 (bs, 1H, COOH), 13.54 (bs, 1H, H-1)

Beispiel 16

3-(5-Ethoxycarbonyl-2-furyl)-indazol (Ausgangsmaterial)

1.75 g (7.7 mmol) 3-(5-Carboxy-2-furyl)-indazol wurden zusammen mit 2.5 ml konzentrierter Schwefelsäure in 60 ml Toluol und 20 ml Ethanol am Wasserabscheider erhitzt. Übliche Aufarbeitung ergab 1.2 g (60 %) der Titelverbindung.
$^1$H-NMR ($D_6$-DMSO): $\delta$ 1.36 (t, 3H, $CH_3$), 4.35 (q, 2H, $CH_2$), 7.10 (d, 1H, H-3'), 7.30 (t, 1H, H-5), 7.44 (t, 1H, H-6), 7.46 (d, 1 H, H-4'), 7.64 (d, 1H, H-7), 8.13 (d, 1H, H-4), 13.60 (bs, 1H, H-1)

Beispiel 17

1-((5-Chlor-2-thienyl)-methyl)-3-(5-ethoxycarbonyl-2-furyl)-indazol (Ausgangsmaterial)

600 mg (2.3 mmol) 3-(5-Ethoxycarbonyl-2-furyl)-indazol wurden zusammen mit 265 mg (2.4 mmol) Kalium-tert-butylat in 10 ml DMF vorgelegt, 430 mg (2.6 mmol) 2-Chlor-5-(chlormethyl)-thiophen wurden in 1 ml DMF zugetropft und es wurde 2 h bei RT gerührt. Übliche Aufarbeitung ergab 350 mg (41 %) der Titelverbindung.
Schmp.: 124-126 °C

Beispiel 19

1-Benzyl-3-(5-chlorcarbonyl-2-furyl)-indazol (Ausgangsmaterial)

15 g (0.05 mol) 1-Benzyl-3-(5-carboxy-2-furyl)-indazol und 36.43 g (0.31 mol) Thionylchlorid wurden in 400 ml Benzol 3 h unter Rückfluß erhitzt. Das abgekühlte Gemisch wurde filtriert und eingeengt. Es resultierten 11.95 g (ca. 76 %) der Titelverbindung, die ohne weitere Reinigung für Folgereaktionen verwendet wurden.

Beispiel 20

1-Benzyl-3-(5-(N-(4-trifluormethyl-phenyl)-carbamoyl)-2-furyl)-indazol

0.29 g (1.8 mmol) 4-Trifluormethylanilin wurden in 20 ml THF vorgelegt, 0.14 g (1.8 mmol) Pyridin wurden zugefügt und bei RT wurde eine Lösung von 0.5 g (1.5 mmol) 1-Benzyl-3-(5-chlorcarbonyl-2-furyl)-indazol in 10 ml THF zugetropft. Nach 0.5 h wurde der Ansatz auf Eiswasser gegossen, der Niederschlag abgesaugt und aus Isopropanol umkristallisiert. Man erhielt 0.47 g (69 %) der Titelverbindung.
Schmp.: 205-206 °C

Analog Beispiel 20 wurden erhalten:

Beispiel 21

1-Benzyl-3-(5-(N-phenyl-carbamoyl)-2-furyl)-indazol

Schmp.: 138-143 °C

Beispiel 22

1-Benzyl-3-(5-(N-(4-chlorphenyl)-carbamoyl)-2-furyl)-indazol

Schmp.: 202-205 °C

Beispiel 23

1-Benzyl-3-(5-(N-(4-nitrophenyl)-carbamoyl)-2-furyl)-indazol

Schmp.: 204-208 °C

Beispiel 24

1-Benzyl-3-(5-(N-(2-naphthyl)-carbamoyl)-2-furyl)-indazol

Schmp.: 167 °C

Beispiel 25

1-Benzyl-3-(5-(N-(2-thiazolyl)-carbamoyl)-2-furyl)-indazol

Schmp.: 167-168 °C

Beispiel 26

1-Benzyl-3-(5-(N-(benzyloxy)-carbamoyl)-2-furyl)-indazol

Schmp.: 140-143 °C

Beispiel 27

1-Benzyl-3-(5-(N-methyl-N-phenyl-carbamoyl)-2-furyl)-indazol

Schmp.: 190 °C

Beispiel 28

1-Benzyl-3-(5-(N-(4-methoxyphenyl)-carbamoyl)-2-furyl)-indazol

Schmp.: 175°C

Beispiel 29

1-Benzyl-3-(5-(N-(2-hydroxyphenyl)-carbamoyl)-2-furyl)-indazol

Schmp.: 196-197 °C

Beispiel 30

1-Benzyl-3-(5-(N-(2-hydroxyethyl)-carbamoyl)-2-furyl)-indazol

0.23 g (3.7 mmol) Ethanolamin wurden in 20 ml THF vorgelegt und eine Lösung von 0.5 g (1.5 mmol) 1-Benzyl-3-(5-chlorcarbonyl-2-furyl)-indazol in 10 ml THF wurde zugetropft. Nach 0.75 h wurde auf Eiswasser gegossen, der Niederschlag abgesaugt und aus Essigester umkristallisiert. Man erhielt 0.33 g (62 %) der Titelverbindung.
Schmp.: 119-127 °C

Analog Beispiel 30 wurden erhalten:

Beispiel 31

1-Benzyl-3-(5-(N-isopropyl-carbamoyl)-2-furyl)-indazol

Schmp.: 156 °C

Beispiel 32

1-Benzyl-3-(5-(N-(n-propyl)-carbamoyl)-2-furyl)-indazol

Schmp.: 152 °C

Beispiel 33

1-Benzyl-3-(5-(N-cyclohexyl-carbamoyl)-2-furyl)-indazol

Schmp.: 165 °C

Beispiel 34

1-Benzyl-3-(5-(N-benzyl-carbamoyl)-2-furyl)-indazol

Schmp.: 113-124 °C

Beispiel 35

1-Benzyl-3-(5-(N-(2-dimethylamino-ethyl)-carbamoyl)-2-furyl)-indazol

Schmp.: 130 °C

Beispiel 36

1-Benzyl-3-(5-(N-(2-diisopropylamino-ethyl)-carbamoyl)-2-furyl)-indazol

Schmp.: 119-127 °C

Beispiel 37

1-Benzyl-3-(5-(N-(carbamoylmethyl)-carbamoyl)-2-furyl)-indazol

Schmp.: 219-222 °C

Beispiel 38

1-Benzyl-3-(5-(N-(2-pyridyl-methyl)-carbamoyl)-2-furyl)-indazol

Schmp.: 160 °C

Beispiel 39

1-Benzyl-3-(5-carbamoyl-2-furyl)-indazol

In 25 ml einer gesättigten Lösung von Ammoniak in THF wurde unter gleichzeitigem Einleiten von Ammoniak eine Lösung von 0.8 g (2.4 mmol) 1-Benzyl-3-(5-chlorcarbonyl-2-furyl)-indazol in 10 ml THF getropft. Nach 45 min wurde der Ansatz auf Eiswasser gegossen, mit Essigester extrahiert, die organische Phase mit 1 N Salzsäure gewaschen, getrocknet und einrotiert. Das Rohprodukt wurde aus Isopropanol umkristallisiert. Man erhielt 0.29 g (38 %) der Titelverbindung.
Schmp.: 259 °C

Beispiel 40

1-Benzyl-3-(5-(N-methyl-carbamoyl)-2-furyl)-indazol

2.97 ml (5.94 mmol) einer 2 M Lösung von Methylamin in THF wurden in 20 ml THF vorgelegt und es wurde bei RT eine Lösung von 0.8 g (2.4 mmol) 1-Benzyl-3-(5-chlorcarbonyl-2-furyl)-indazol in 10 ml THF zugetropft. Nach 1 h wurde der Ansatz auf Eiswasser gegossen und das sich abscheidende Öl mit Essigester extrahiert. Die organische Phase wurde mit 1 N Salzsäure gewaschen, getrocknet und einrotiert. Das Rohprodukt wurde aus Isopropanol umkristallisiert. Man erhielt 0.44 g (55 %) der Titelverbindung.
Schmp.: 182-183 °C

Beispiel 46

1-Benzyl-3-(5-methoxycarbonyl-2-furyl)-5-methyl-pyrazol und Isomer (Ausgangsmaterial)

46a) 5-(1,3-Dioxo-butyl)-furan-2-carbonsäuremethylester

Zu einer auf 0 °C gekühlten Mischung von 31.5 g (0.25 mol) Furan-2-carbonsäuremethylester und 102 g (1 mol) Acetanhydrid wurden innerhalb 90 min 130 g (0.5 mol) Zinn-(IV)-chlorid zugetropft. Nach 16 h Rühren bei RT wurde airf 0 °C gekühlt, 20 ml 30 %ige Salzsäure wurden zugetropft und es wurde anschließend 3 h bei RT nachgerührt. Das resultierende Gemisch wurde auf 200 ml Wasser gegossen und mehrmals mit Essigester extrahiert. Die vereinigten organischen Extrakte wurden mit Wasser gewaschen, getrocknet und eingeengt. Der Rückstand wurde an Kieselgel mit Dichlormethan chromatographiert. Die Hauptfraktion wurde aus Isopropanol umkristallisiert. Man erhielt 19 g (36 %) der Titelverbindung. Schmp.: 111-112 °C

46b) 1-Benzyl-3-(5-methoxycarbonyl-2-furyl)-5-methyl-pyrazol und Isomer

8 g (38.1 mmol) 5-(1,3-Dioxo-butyl)-furan-2-carbonsäuremethylester, 5.1 g (41.9 mmol) Benzylhydrazin und 1 ml Essigsäure wurden in 150 ml Ethanol 2 h unter Rückfluß erhitzt. Zur Aufarbeitung wurde eingeengt, der Rückstand in Methylenchlorid aufgenommen, die Lösung mit 2 N Natronlauge gewaschen, mit Natriumsulfat getrocknet und eingeengt. Das zurückbleibende Öl kristallisierte über Nacht durch und wurde aus wenig Isopropanol umkristallisiert. Man erhielt 7.95 g (70 %) 1-Benzyl-3-(5-methoxycarbonyl-2-furyl)-5-methyl-pyrazol im Gemisch mit dem isomeren 1-Benzyl-5-(5-methoxycarbonyl-2-furyl)-3-methyl-pyrazol. Schmp.: 65-66 °C

Beispiel 47

1-Benzyl-3-(2-furyl)-5-trifluormethyl-pyrazol (Ausgangsmaterial)

10 g (48.5 mmol) 4,4,4-Trifluor-1-(2-furyl)-1,3-butadion, 7.5 g (53.4 mmol) Benzylhydrazin und 0.5 ml Essigsäure wurden in 75 ml Ethanol 2 h unter Rückfluß erhitzt. Der Ansatz wurde eingeengt, der Rückstand in Dichlormethan gelöst, die Lösung alkalisch extrahiert und anschließend die organische Phase getrocknet und eingeengt. Der Rückstand wurde an Kieselgel mit n-Hexan/Essigester (5:1) gereinigt. Man erhielt 9.0 g (64 %) 1-Benzyl-5-(2-furyl)-3-trifluormethyl-pyrazol neben 4.0 g nicht cyclisiertem Hydrazon. Dieses Hydrazon wurde in 200 ml Methanol gelöst und mit 1 ml konz. Schwefelsäure 2 h unter Rückfluß erhitzt. Anschließend wurde eingeengt, mit Wasser versetzt, mit Dichlormethan extrahiert, die organische Phase getrocknet und erneut eingeengt. Man erhielt 3.1 g (22 %) 1-Benzyl-3-(2-furyl)-5-trifluormethyl-pyrazol.
$^1$H-NMR ($D_6$-DMSO): $\delta$ = 5.51 (s, 2H, $CH_2$), 6.60 (dd, 1H, H-4'), 6.92 (d, 1H, H-3'), 7.10.-7.43 (m, 6H, Phenyl-H, H-4), 7.75 (d, 1H, H-5')

Beispiel 48

1-Benzyl-3-(5-formyl-2-furyl)-5-trifluormethyl-pyrazol (Ausgangsmaterial)

10 ml trockenes DMF wurden bei 10 °C vorgelegt, 1.9 g (12.4 mmol) Phosphoroxychlorid wurden zugetropft und 30 min gerührt. Dazu wurde eine Lösung von 3 g (10.3 mmol) 1-Benzyl-3-(2-furyl)-5-trifluormethyl-pyrazol in 10 ml DMF getropft und über Nacht bei RT gerührt. Der Ansatz wurde auf Eiswasser gegeben und mit Kaliumcarbonatlösung auf pH 9 gestellt. Das ausgefallene Produkt wurde abgesaugt und im Vakuumtrockenschrank bei 50 °C getrocknet. Man erhielt 3.3 g (100 %) der Titelverbindung.
Schmp.: 76.5-77.5 °C

Beispiel 50

3-(5-Carboxy-2-furyl)-1-(4-trifluormethylpyrimidin-2-yl)-indazol-Hydrochlorid (Ausgangsmaterial)

1.3 g (5.7 mmol) 3-(5-Carboxy-2-furyl)-indazol wurden zusammen mit 1.3 g (11.4 mmol) Kalium-tert-butylat in 5 ml trockenem DMF gelöst und mit 2-Chlor-4-trifluormethyl-pyrimidin, gelöst in 5 ml DMF, versetzt. Nach 3 h Rühren bei 60 °C wurde eingeengt, in 500 ml Wasser aufgenommen, mit Essigester extrahiert und das Hydrochlorid aus der wäßrigen Phase durch Zugabe von 1 N Salzsäure abgeschieden. Man erhielt 420 mg (25 %) der Titelverbindung.
Schmp.: 258-261 °C

Pharmakologische Untersuchungen

1 ) Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enyzm-Immuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt.

Die eingesetzte sGC war aus Rinderlunge isoliert worden (siehe Methods in Enzymology, Band 195, S. 377). Die Testlösungen (100 µl pro well) enthielten 50 mM Triethanolamin(TEA)-Puffer (pH 7.5), 3 mM $MgCl_2$, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen das Lösungsmittel. Die Prüfsubstanzen wurden in Dimethylsulfoxid (DMSO) gelöst und die Lösung mit DMSO/Wasser verdünnt, so daß die Endkonzentration an Prüfsubstanz in der Testlösung 50 µM betrug. Die DMSO-Konzentration in der Testlösung betrug 5 % (v/v). Die Reaktion wurde durch Zugabe der sGC gestartet. Der Reaktionsmix wurde für 15 bis 20 Minuten bei 37 °C inkubiert und dann durch Eiskühlung und Zugabe des Stop-Reagenz (50 mM EDTA, pH 8.0) gestoppt. Ein Aliquot von 50 µl wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenz Wellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontrollversuchen (mit Lösungsmittel statt Prüfsubstanz) gefunden wurde (berechnet nach der Formel

$$\text{n-fache Stimulierung} = [\text{cGMP}]_{\text{Prüfsubstanz}} / [\text{cGMP}]_{\text{Kontrolle}} ).$$

Es wurden folgende Werte ermittelt:

| Verbindung | Konzentration | n-fache Stimulierung |
|---|---|---|
| Beispiel 21 | 100 µM | 3-fach |
| Beispiel 25 | 10 µM | 2.8-fach |
| Beispiel 40 | 100 µM | 2.4-fach |

2) Relaxation der Ratten-Aorta

Für diesen Test wurden normotensive, männliche Wistar-Kyoto-Ratten durch einen Schlag auf das Genick getötet. Der Bauchraum und der Brustkorb wurden durch eine mittlere Stemotomie eröffnet. Anschließend wurde die deszendierende Aorta entnommen, von Bindegewebe befreit und in 8 Ringe von ca. 4 mm Länge geteilt. In das Lumen von 4 der 8 Ringe wurde eine Pinzettenspitze eingebracht. Durch vorsichtige, rollende Bewegungen der Ringe über die Pinzettenspitze wurde das Endothel entfernt. Alle 8 Aortenringe (4 mit Endothel und 4 ohne Endothel) wurden anschließend in ein Organbad (Schuler-Organbad; Hugo Sachs Elektronik) mit konstanter Temperatur von 37 °C zur isometrischen Messung des kontraktilen Tonus eingehängt. Die Ringe wurden 30 Minuten bei einer Ruhespannung von 1 g in carbonierter (95 % $O_2$; 5 % $CO_2$) Krebs-Henseleit-Lösung (Zusammensetzung: $Na^+$ 144.0 mM; $K^+$ 5.9 mM; $Cl^-$ 126.9 mM; $Ca^{2+}$ 1.6 mM; $Mg^{2+}$ 1.2 mM; $H_2PO_4^-$ 1.2 mM; $SO_4^{2-}$ 1.2 mM; $HCO_3^-$ 25.0 mM; D-Glucose 11.1 mM) vom pH-Wert 7.4 kalibriert. Außerdem wurde der Krebs-Henseleit-Lösung 1 µmol/l Indomethacin zur Hemmung der Prostaglandin-Biosynthese zugegeben. Anschließend wurden die Ringe durch Zugabe von Phenylephrin (Konzentration in der Lösung: 1 µM) vorkontrahiert und die endothelabhängige Relaxation bzw. der funktionelle Verlust des Endothels wird durch Gabe von Acetylcholin (Konzentration in der Lösung: 1 µM) getestet. Im Anschluß an eine 30-minütige Waschperiode wurden die Ringe dann erneut durch Zusatz von Phenylephrin (1 µM) vorkontrahiert und durch Gabe von kumulativen Dosen der Prüfsubstanzen der Formel I wurde deren relaxierende Wirkung bestimmt. Die Auswertung der Daten erfolgte nach Standardverfahren. Angegeben wird die Konzentration $IC_{50}$, durch die die Kontraktion um 50 % gehemmt wird (50 %ige Relaxation).

Es wurden folgende Werte ermittelt:

| Verbindung | | $IC_{50}$ |
|---|---|---|
| Beispiel 40 | Ring mit Endothel | 2.4 µM |
| Beispiel 40 | Ring ohne Endothel | 0.2 µM |

**Patentansprüche**

**1.** Verbindungen der Formel I,

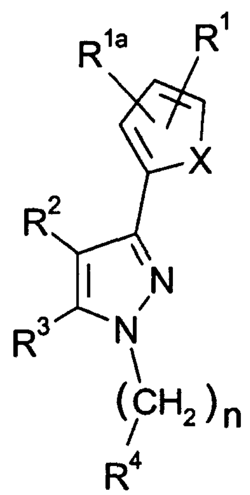

I

in der

X für O, S, NH oder $N(CH_3)$ steht;
$R^1$ für CO-$NR^{11}R^{12}$ steht;
$R^{1a}$ für Wasserstoff steht;
$R^2$ und $R^3$ unabhängig voneinander für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het oder den Rest Het-$(C_1-C_4)$-alkyl stehen, wobei für $R^2$ oder $R^3$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere Substituenten $R^5$ substituiert sein können, oder $R^2$ und $R^3$ zusammen mit den sie tragenden Kohlenstoffatomen einen 5- bis 7-gliedrigen carbocyclischen Ring bilden, der eine oder mehrere Doppelbindungen enthalten kann und der unsubstituiert oder durch einen oder mehrere Substituenten
$R^5$ substituiert sein kann;
$R^4$ für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het oder den Rest Het-$(C_1-C_4)$-alkyl stehen, wobei für $R^4$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere Substituenten $R^5$ substituiert sein können, und wobei, wenn n = 0 ist, $R^4$ nicht für Wasserstoff stehen kann;
n für 0, 1 oder 2 steht;
Het für einen 5- bis 7-gliedrigen, gesättigten oder ungesättigten Heterocyclus steht;
$R^5$ für Halogen, $(C_1-C_5)$-Alkyl, $OR^6$, $NR^7R^8$, CO-$OR^9$, CO-$R^{10}$, CO-$NR^{11}R^{12}$, CO-$NR^{12}$-$OR^{11}$, $S(O)_m$-$R^{13}$, $S(O)_2$-$NR^{14}R^{15}$, $NO_2$, CN oder $CF_3$ steht, wobei mehrfach auftretende Reste $R^5$ unabhängig voneinander sind und gleich oder verschieden sein können;
$R^6$, $R^7$, $R^8$, $R^{11}$ und $R^{14}$ unabhängig voneinander für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het, den Rest Het-$(C_1-C_4)$-alkyl, CO-$R^{16}$ oder $S(O)_2$-$R^{17}$ stehen, wobei für $R^6$, $R^7$, $R^8$, $R^{11}$ und $R^{14}$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere gleiche oder verschiedene Substituenten aus der Reihe Halogen, $(C_1-C_5)$-Alkyl, $OR^{18}$, $NR^{19}R^{20}$, CO-$OR^{21}$, CO-$R^{22}$, CO-$NR^{23}R^{24}$, CO-$NR^{24}$-$OR^{23}$, $S(O)_m$-$R^{25}$, $S(O)_2$-$NR^{26}R^{27}$, $NO_2$, CN und $CF_3$ substituiert sein können, und wobei mehrfach auftretende Reste $R^6$, $R^7$, $R^8$, $R^{11}$ und $R^{14}$ unabhängig voneinander sind und gleich oder verschieden sein können;
$R^9$, $R^{10}$, $R^{12}$, $R^{13}$ und $R^{15}$ unabhängig voneinander für Wasserstoff, $(C_1-C_{10})$-Alkyl, $(C_6-C_{14})$-Aryl, $(C_6-C_{14})$-Aryl-$(C_1-C_4)$-alkyl, $(C_3-C_7)$-Cycloalkyl, $(C_3-C_7)$-Cycloalkyl-$(C_1-C_4)$-alkyl, den Rest Het oder den Rest Het-$(C_1-C_4)$-alkyl stehen, wobei für $R^9$, $R^{10}$, $R^{12}$, $R^{13}$ und $R^{15}$ stehende Alkylreste, Arylreste, Arylalkylreste, Cycloalkylreste, Cycloalkyl-alkylreste, Reste Het und Reste Het-alkyl jeweils unsubstituiert oder durch einen oder mehrere gleiche oder verschiedene Substituenten aus der Reihe Halogen, $(C_1-C_5)$-Alkyl, $OR^{18}$, $NR^{19}R^{20}$, CO-$OR^{21}$, CO-$R^{22}$, CO-$NR^{23}R^{24}$, CO-$NR^{24}$-$OR^{23}$, $S(O)_m$-$R^{25}$, $S(O)_2$-$NR^{26}R^{27}$, $NO_2$, CN und $CF_3$ substituiert sein können, und wobei mehrfach auftretende Reste $R^9$, $R^{10}$, $R^{12}$, $R^{13}$ und $R^{15}$ unabhängig voneinander sind und gleich oder verschieden sein können;
oder die zwei Reste $R^7$ und $R^8$, die zwei Reste $R^{11}$ und $R^{12}$ und die zwei Reste $R^{14}$ und $R^{15}$, jeweils zusammen mit dem die zwei Reste tragenden Stickstoffatom, einen 5- bis 7-gliedrigen, gesättigten oder ungesättigten heterocyclischen Ring bilden, der noch ein zusätzliches Ring-Heteroatom aus der Reihe Stickstoff, Sauerstoff und Schwefel enthalten kann und der durch einen oder mehrere gleiche oder verschiedene Substituenten aus der Reihe $(C_1-C_4)$-Alkyl und Halogen substituiert sein kann;
$R^{16}$ für Wasserstoff, $(C_1-C_6)$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het oder den Rest Het-$(C_1-C_4)$-alkyl steht;
$R^{17}$ für $(C_1-C_6)$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het oder den Rest Het-$(C_1-C_4)$-alkyl steht;
$R^{18}$, $R^{19}$, $R^{20}$, $R^{23}$ und $R^{26}$ unabhängig voneinander für Wasserstoff, $(C_1-C_{10})$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het, den Rest Het-$(C_1-C_4)$-alkyl, CO-$R^{16}$ oder $S(O)_2$-$R^{17}$ stehen, wobei mehrfach auftretende Reste $R^{18}$, $R^{19}$, $R^{20}$, $R^{23}$ und $R^{26}$ unabhängig voneinander sind und gleich oder verschieden sein können;
$R^{21}$, $R^{22}$, $R^{24}$, $R^{25}$ und $R^{27}$ unabhängig voneinander für Wasserstoff, $(C_1-C_{10})$-Alkyl, $(C_3-C_7)$-Cycloalkyl, $(C_3-C_7)$-Cycloalkyl-$(C_1-C_4)$-alkyl, $(C_6-C_{14})$-Aryl, $(C_6-C_{14})$-Aryl-$(C_1-C_4)$-alkyl, den Rest Het oder den Rest Het-$(C_1-C_4)$-alkyl stehen, wobei mehrfach auftretende Reste $R^{21}$, $R^{22}$, $R^{24}$, $R^{25}$ und $R^{27}$ unabhängig voneinander sind und gleich oder verschieden sein können;
oder die zwei Reste $R^{19}$ und $R^{20}$, die zwei Reste $R^{23}$ und $R^{24}$ und die zwei Reste $R^{26}$ und $R^{27}$, jeweils zusammen mit dem die zwei Reste tragenden Stickstoffatom, einen 5- bis 7-gliedrigen, gesättigten oder ungesättigten heterocyclischen Ring bilden, der noch ein zusätzliches Ring-Heteroatom aus der Reihe Stickstoff, Sauerstoff und Schwefel enthalten kann und der durch einen oder mehrere gleiche oder verschiedene Substituenten aus der

Reihe ($C_1$-$C_4$)-Alkyl und Halogen substituiert sein kann;
m für 0, 1 oder 2 steht;
in allen ihren stereoisomeren Formen und Mischungen davon in allen Verhältnissen, und ihre physiologisch verträglichen Salze.

**2.** Verbindungen der Formel I gemäß Anspruch 1, in der $R^2$ und $R^3$ zusammen mit. den sie tragenden Kohlenstoffatomen einen Benzolring bilden, der unsubstituiert oder durch einen oder mehrere gleiche oder verschiedene Substituenten $R^5$ substituiert sein kann, in allen ihren stereoisomeren Formen und Mischungen davon in allen Verhältnissen, und ihre physiologisch verträglichen Salze.

**3.** Verbindungen der Formel I gemäß Anspruch 1 und/oder 2, in der X für O oder S steht, in allen ihren stereoisomeren Formen und Mischungen davon in allen Verhältnissen, und ihre physiologisch verträglichen Salze.

**4.** Verbindungen der Formel I gemäß einem oder mehreren der Ansprüche 1 bis 3, in der $R^{11}$ für Wasserstoff steht und $R^{12}$ unsubstituiertes ($C_1$-$C_4$)-Alkyl, ($C_1$-$C_4$)-Alkyl, das durch einen Rest aus der Reihe $OR^{18}$, $NR^{19}R^{20}$ und $CO\text{-}NR^{23}R^{24}$ substituiert ist, 5- oder 6-gliedriges Heteroaryl, unsubstituiertes Phenyl oder Phenyl, das durch einen, zwei oder drei gleiche oder verschiedene Reste aus der Reihe Halogen, ($C_1$-$C_5$)-Alkyl, $OR^{18}$, $NR^{19}R^{20}$, $CO\text{-}OR^{21}$, $CO\text{-}R^{22}$, $CO\text{-}NR^{23}R^{24}$, $S(O)_m\text{-}R^{25}$, $S(O)_2\text{-}NR^{26}R^{27}$, $NO_2$, CN und $CF_3$ substituiert ist, steht, in allen ihren stereoisomeren Formen und Mischungen davon in allen Verhältnissen, und ihre physiologisch verträglichen Salze.

**5.** Verfahren zur Herstellung von Verbindungen der Formel I gemäß einem oder mehreren der Ansprüche 1 bis 4, **dadurch gekennzeichnet, daß** man

a) 1,3-Dicarbonylverbindungen der Formel II mit Hydrazinen der Formel III oder deren Salzen umsetzt,

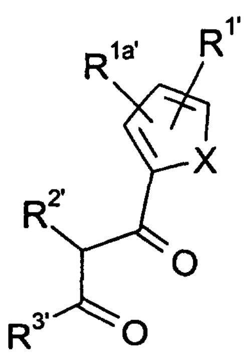

II

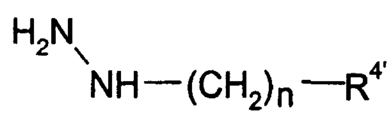

III

wobei in den Formeln II und III die Reste X, $R^{1'}$, $R^{1a'}$ und $R^{4'}$ und die Zahl n die in den Ansprüchen 1 bis 4 angegebenen Bedeutungen von X, $R^1$, $R^{1a}$, $R^4$ und n haben und die Reste $R^{2'}$ und $R^{3'}$ die in den Ansprüchen 1 bis 4 angegebenen Bedeutungen von $R^2$ und $R^3$ haben, aber nicht zusammen mit den sie tragenden Kohlenstoffatomen einen aromatischen Ring bilden können, und zusätzlich in den Resten $R^{1'}$, $R^{1a'}$, $R^{2'}$, $R^{3'}$ und $R^{4'}$ funktionelle Gruppen in geschützter Form oder in Form von Vorstufen vorliegen können, und anschließend gegebenenfalls die Reste $R^{1'}$, $R^{1a'}$, $R^{2'}$, $R^{3'}$ und $R^{4'}$ in die Reste $R^1$, $R^{1a}$, $R^2$, $R^3$ und $R^4$ mit den in den Ansprüchen 1 bis 4 angegebenen Bedeutungen überführt; oder

b) Verbindungen der Formel VII mit Hydrazinen der Formel III oder deren Salzen umsetzt,

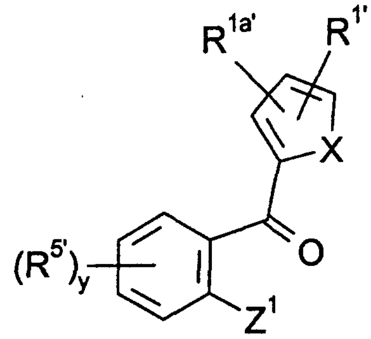

VII

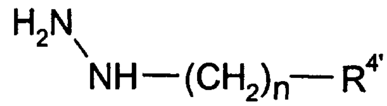

III

wobei in den Formeln VII und III die Reste X, $R^{1'}$, $R^{1a'}$ und $R^{4'}$ und die Zahl n die unter a) angegebenen Bedeutungen haben, $R^{5'}$ die in den Ansprüchen 1 bis 4 angegebenen Bedeutungen von $R^5$ hat und zusätzlich in den Resten $R^{5'}$ funktionelle Gruppen in geschützter Form oder in Form von Vorstufen vorliegen können, y für 0, 1, 2, 3 oder 4 steht und $Z^1$ für eine Abgangsgruppe steht, und anschließend gegebenenfalls die Reste $R^{1'}$, $R^{1a'}$, $R^{4'}$ und $R^{5'}$ in die Reste $R^1$, $R^{1a}$, $R^4$ und $R^5$ mit den in den Ansprüchen 1 bis 4 angegebenen Bedeutungen überführt; oder

c) Verbindungen der Formel VII mit Verbindungen der Formel IX unter Abspaltung der Acylgruppe R-CO zu Verbindungen der Formel X umsetzt und diese mit Verbindungen der Formel XI alkyliert,

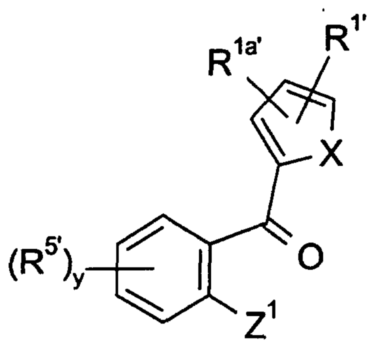

VII

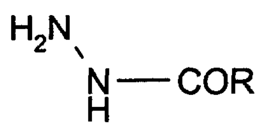

IX

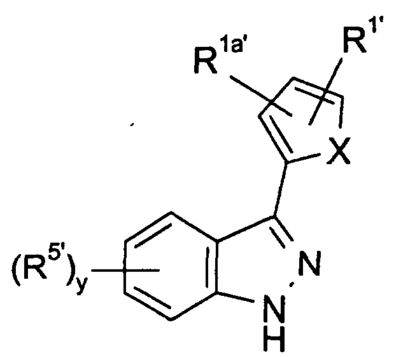

X

$$R^{4'}-(CH_2)_n-Z^2$$

XI

wobei in den Formeln VII, IX, X und XI die Reste X, $R^{1'}$, $R^{1a'}$, $R^{4'}$ und $R^{5'}$, $Z^1$, n und y die unter a) und b) angegebenen Bedeutungen haben, $Z^2$ für eine Abgangsgruppe steht und R für einen Alkylrest oder Arylrest steht, und anschließend gegebenenfalls die Reste $R^{1'}$, $R^{1a'}$, $R^{4'}$ und $R^{4'}$ in die Reste $R^1$, $R^{1a}$, $R^4$ und $R^5$ mit den in den Ansprüchen 1 bis 4 angegebenen Bedeutungen überführt.

**6.** Verbindungen der Formel I gemäß einem oder mehreren der Ansprüche 1 bis 4 und/oder ihre physiologisch verträglichen Salze zur Verwendung als Arzneimittel.

**7.** Pharmazeutisches Präparat, **dadurch gekennzeichnet, daß** es eine oder mehrere Verbindungen der Formel I gemäß einem oder mehreren der Ansprüche 1 bis 4 und/oder ihre physiologisch verträglichen Salze neben pharmazeutisch einwandfreien Trägerstoffen und/oder Zusatzstoffen enthält.

8. Verwendung von Verbindungen der Formel I gemäß einem oder mehreren der Ansprüche 1 bis 4 und/oder ihren physiologisch verträglichen Salzen zur Herstellung eines Medikaments zur Aktivierung der löslichen Guanylatcyclase.

9. Verwendung von Verbindungen der Formel I gemäß einem oder mehreren der Ansprüche 1 bis 4 und/oder ihren physiologisch verträglichen Salzen zur Herstellung eines Medikaments zur Therapie oder Prophylaxe von Herz-Kreislauf-Erkrankungen, endothelialer Dysfunktion, diastolischer Dysfunktion, Atherosklerose, Bluthochdruck, Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz, Diabetes oder Leberzirrhose oder zur Verbesserung einer eingeschränkten Lernfähigkeit oder Gedächtnisleistung.

## Claims

1. A compound of the formula I

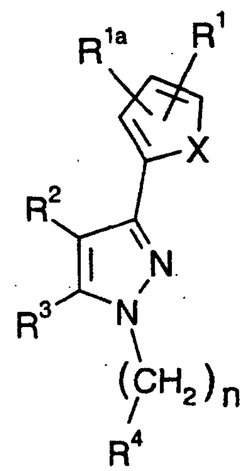

I

in which
X is O, S, NH or $N(CH_3)$;
$R^1$ is $CO-NR^{11}R^{12}$;
R1 a is hydrogen;
$R^2$ and $R^3$ independently of one another are hydrogen, $(C_1-C_{10})$-alkyl, $(C_6-C_{14})$-aryl, $(C_6-C_{14})$-aryl-$(C_1-C_4)$-alkyl, $(C_3-C_7)$-cycloalkyl, $(C_3-C_7)$-cycloalkyl-$(C_1-C_4)$-alkyl, the radical Het or the radical Het-$(C_1-C_4)$-alkyl, where alkyl radicals, aryl radicals, arylalkyl radicals, cycloalkyl radicals and cycloalkylalkyl radicals which are represented by $R^2$ or $R^3$ and the radicals Het and Het-alkyl may in each case be unsubstituted or substituted by one or more substituents $R^5$,
or $R^2$ and $R^3$ together with the carbon atoms which carry them form a 5- to 7-membered carbocyclic ring which may contain one or more double bonds and which may be unsubstituted or substituted by one or more substituents $R^5$;
$R^4$ is hydrogen, $(C_1-C_{10})$-alkyl, $(C_6-C_{14})$-aryl, $(C_6-C_{14})$-aryl-$(C_1-C_4)$-alkyl, $(C_3-C_7)$-cycloalkyl, $(C_3-C_7)$-cycloalkyl-$(C_1-C_4)$-alkyl, the radical Het or the radical Het-$(C_1-C_4)$-alkyl, where alkyl radicals, aryl radicals, arylalkyl radicals, cycloalkyl radicals and cycloalkylalkyl radicals which are represented by $R^4$ and the radicals Het and Het-alkyl may in each case be unsubstituted or substituted by one or more substituents $R^5$, and where, if n = 0, $R^4$ may not be hydrogen;
n is 0, 1 or 2;
Het is a 5- to 7-membered, saturated or unsaturated heterocycle;
$R^5$ is halogen, $(C_1-C_5)$-alkyl, $OR^6$, $NR^7R^8$, $CO-OR^9$, $CO-R^{10}$, $CO-NR^{11}R^{12}$, $CO-NR^{12}-OR^{11}$, $S(O)_m-R^{13}$, $S(O)_2-NR^{14}R^{15}$, $NO_2$, CN or $CF_3$, where radicals $R^5$ that occur repeatedly are independent of one another and may b.e identical or different;

$R^6$, $R^7$, $R^8$, $R^{11}$ and $R^{14}$ independently of one another are hydrogen, $(C_1-C_{10})$-alkyl, $(C_6-C_{14})$-aryl, $(C_6-C_{14})$-aryl-$(C_1-C_4)$-alkyl, $(C_3-C_7)$-cycloalkyl, $(C_3-C_7)$-cycloalkyl-$(C_1-C_4)$-alkyl, the radical Het, the radical Het-$(C_1-C_4)$-alkyl, CO-$R^{16}$ or $S(O)_2$-$R^{17}$, where alkyl radicals, aryl radicals, arylalkyl radicals, cycloalkyl radicals and cycloalkylalkyl radicals which are represented by $R^6$, $R^7$, $R^8$, $R^{11}$ and $R^{14}$ and the radicals Het and Het-alkyl may in each case be unsubstituted or substituted by one or more identical or different substituents selected from the group consisting of halogen, $(C_1-C_5)$-alkyl, $OR^{18}$, $NR^{19}R^{20}$, CO-$OR^{21}$, CO-$R^{22}$, CO-$NR^{23}R^{24}$, CO-$NR^{24}$-$OR^{23}$, $S(O)_m$ $R^{25}$, $S(O)_2$-$NR^{26}R^{27}$, $NO_2$, CN and $CF_3$, and where radicals $R^6$, $R^7$, $R^8$, $R^{11}$ and $R^{14}$ that occur repeatedly are independent of one another and may be identical or different;

$R^9$, $R^{10}$, $R^{12}$, $R^{13}$ and $R^{15}$ independently of one another are hydrogen, $(C_1-C_{10})$-alkyl, $(C_6-C_{14})$-aryl, $(C_6-C_{14})$-aryl-$(C_1-C_4)$-alkyl, $(C_3-C_7)$-cycloalkyl, $(C_3-C_7)$-cycloalkyl-$(C_1-C_4)$-alkyl, the radical Het or the radical Het-$(C_1-C_4)$-alkyl, where alkyl radicals, aryl radicals, arylalkyl radicals, cycloalkyl radicals and cycloalkylalkyl radicals which are represented by $R^9$, $R^{10}$, $R^{12}$, $R^{13}$ and $R^{15}$ and the radicals Het and Het-alkyl may in each case be unsubstituted or substituted by one or more identical or different substituents selected from the group consisting of halogen, $(C_1-C_5)$-alkyl, $OR^{18}$, $NR^{19}R^{20}$, CO-$OR^{21}$, CO-$R^{22}$, CO-$NR^{23}R^{24}$, CO-$NR^{24}$-$OR^{23}$, $S(O)_m$-$R^{25}$, $S(O)_2$-$NR^{26}R^{27}$, $NO_2$, CN and $CF_3$, and where radicals $R^9$, $R^{10}$, $R^{12}$, $R^{13}$ and $R^{15}$ that occur repeatedly are independent of one another and may be identical or different;

or the two radicals $R^7$ and $R^8$, the two radicals $R^{11}$ and $R^{12}$ and the two radicals $R^{14}$ and $R^{15}$, in each case together with the nitrogen atom which carries the two radicals, form a 5- to 7-membered, saturated or unsaturated heterocyclic ring which may contain an additional ring heteroatom selected from the group consisting of nitrogen, oxygen and sulfur and which may be substituted by one or more identical or different ° substituents selected from the group consisting of $(C_1-C_4)$-alkyl and halogen;

$R^{16}$ is hydrogen, $(C_1-C_6)$-alkyl, $(C_3-C_7)$-cycloalkyl, $(C_3-C_7)$-cycloalkyl-$(C_1-C_4)$-alkyl, $(C_6-C_{14})$-aryl, $(C_6-C_{14})$-aryl-$(C_1-C_4)$-alkyl, the radical Het or the radical Het-$(C_1-C_4)$-alkyl;

$R^{17}$ is $(C_1-C_6)$-alkyl, $(C_3-C_7)$-cycloalkyl, $(C_3-C_7)$-cycloalkyl-$(C_1-C_4)$-alkyl, $(C_6-C_{14})$-aryl, $(C_6-C_{14})$-aryl-$(C_1-C_4)$-alkyl, the radical Het or the radical Het-$(C_1-C_4)$-alkyl;

$R^{18}$, $R^{19}$, $R^{20}$, $R^{23}$ and $R^{26}$ independently of one another are hydrogen, $(C_1-C_{10})$-alkyl, $(C_3-C_7)$-cycloalkyl, $(C_3-C_7)$-cycloalkyl-$(C_1-C_4)$-alkyl, $(C_6-C_{14})$-aryl, $(C_6-C_{14})$-aryl-$(C_1-C_4)$-alkyl, the radical Het, the radical Het- $(C_1-C_4)$-alkyl, CO-$R^{16}$ or $S(O)_2$-$R^{17}$, where radicals $R^{18}$, $R^{19}$, $R^{20}$, $R^{23}$ and $R^{26}$ that occur repeatedly are independent of one another and may be identical or different;

$R^{21}$, $R^{22}$, $R^{24}$, $R^{25}$ and $R^{27}$ independently of one another are hydrogen, $(C_1-C_{10})$-alkyl, $(C_3-C_7)$-cycloalkyl, $(C_3-C_7)$-cycloalkyl-$(C_1-C_4)$-alkyl, $(C_6-C_{14})$-aryl, $(C_6-C_{14})$-aryl-$(C_1-C_4)$-alkyl, the radical Het or the radical Het-$(C_1-C_4)$-alkyl, where radicals $R^{21}$, $R^{22}$, $R^{24}$, $R^{25}$ and $R^{27}$ that occur repeatedly are independent of one another and may be identical or different; or the two radicals $R^{19}$ and $R^{20}$, the two radicals $R^{23}$ and $R^{24}$ and the two radicals $R^{26}$ and $R^{27}$, in each case together with the nitrogen atom which carries the two radicals, form a 5- to 7-membered, saturated or unsaturated heterocyclic ring which may contain an additional ring heteroatom selected from the group consisting of nitrogen, oxygen and sulfur and which may be substituted by one or more identical or different substituents selected from the group consisting of $(C_1-C_4)$-alkyl and halogen;

m is 0, 1 or 2;

in all its stereoisomer forms and mixtures thereof in all ratios, and also its physiologically acceptable salts.

**2.** The compound of the formula I as claimed in claim 1, in which $R^2$ and $R^3$ together with the carbon atoms which carry them form a benzene ring which may be unsubstituted or substituted by one or more identical or different substituents $R^5$, in all its stereoisomeric forms and mixtures thereof in all ratios, and also its physiologically acceptable salts.

**3.** The compound of the formula I as claimed in claim 1 and/or 2, in which X is O or S, in all its stereoisomeric forms and mixtures thereof in all ratios, and also its physiologically acceptable salts.

**4.** The compound of the formula I as claimed in one or more of claims 1 to 3, in which $R^{11}$ is hydrogen and $R^{12}$ is unsubstituted $(C_1-C_4)$-alkyl, $(C_1-C_4)$-alkyl which is substituted by a radical selected from the group consisting of $OR^{18}$, $NR^{19}R^{20}$ and CO-$NR^{23}R^{24}$, 5- or 6-membered heteroaryl, unsubstituted phenyl or phenyl which is substituted by one, two or three identical or different radicals selected from the group consisting of halogen, $(C_1-C_5)$-alkyl, $OR^{18}$, $NR^{19}R^{20}$, CO-$OR^{21}$, CO-$R^{22}$, CO-$NR^{23}R^{24}$, $S(O)_m$-$R^{25}$, $S(O)_2$-$NR^{26}R^{27}$, $NO_2$, CN and $CF_3$, in all its stereoisomeric forms and mixtures thereof in all ratios, and also its physiologically acceptable salts.

**5.** A process for preparing compounds of the formula I as claimed in one or more of claims 1 to 4, which comprises

a) reacting 1,3-dicarbonyl compounds of the formula II with hydrazines of the formula III or salts thereof,

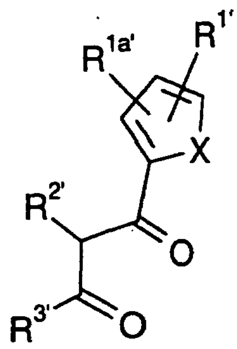

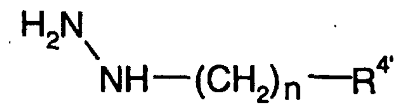

III

II

where in the formulae II and III the radicals X, $R^{1'}$, $R^{1a'}$ and $R^{4'}$ and the number n have the meanings of X, $R^1$, $R^{1a}$, $R^4$ and n given in claims 1 to 4 and the radicals $R^{2'}$ and $R^{3'}$ have the meanings of $R^2$ and $R^3$ given in claims 1 to 4, but they may not form an aromatic ring together with the carbon atoms which carry them, and where additionally in the radicals $R^{1'}$, $R^{1a'}$, $R^{2'}$, $R^{3'}$ and $R^{4'}$ functional groups may be present in protected form or in the form of precursors, and subsequently, if appropriate, converting the radicals $R^{1'}$, $R^{1a'}$, $R^{2'}$, $R^{3'}$ and $R^{4'}$ into the radicals $R^1$, $R^{1a}$, $R^2$, $R^3$ and $R^4$ which have the meanings given in claims 1 to 4; or

b) reacting compounds of the formula VII with hydrazines of the formula III or salts thereof

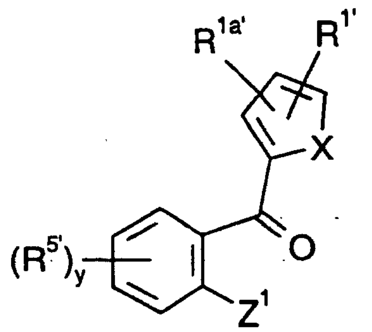

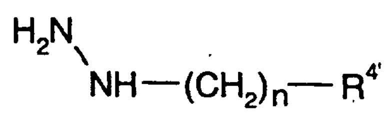

III

VII

where in the formulae VII and III the radicals X, $R^{1'}$, $R^{1a'}$ and $R^{4'}$ and the number n have the meanings given under a), $R^{5'}$ has the meanings of $R^5$ given in claims 1 to 4 and where additionally in the radicals $R^{5'}$ functional groups may be present in protected form or in the form of precursors, y is 0, 1, 2, 3 or 4 and $Z^1$ is a leaving group, and subsequently, if appropriate, converting the radicals $R^{1'}$, $R^{1a'}$, $R^{4'}$ and $R^{5'}$ into the radicals $R^1$, $R^{1a}$, $R^4$ and $R^5$ which have the meanings given in claims 1 to 4; or

c) reacting compounds of the formula VII with compounds of the formula IX with elimination of the acyl group R-CO to give compounds of the formula X, and alkylating these with compounds of the formula XI

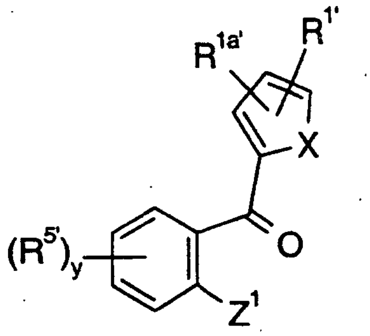

VII

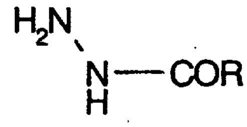

IX

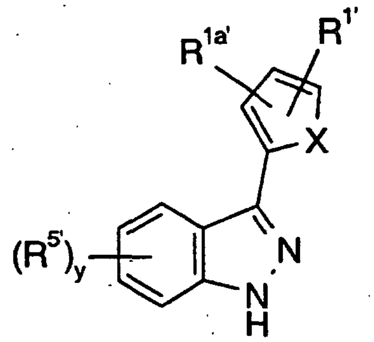

X

$$R^{4'}-(CH_2)_n-Z^2$$

XI

where in the formulae VII, IX, X and XI the radicals X, $R^{1'}$, $R^{1a'}$, $R^{4'}$ and $R^{5'}$, $Z^1$, n and y have the meanings given under a) and b), $Z^2$ is a leaving group and R is an alkyl radical or aryl radical, and subsequently, if appropriate, converting the radicals $R^{1'}$, $R^{1a'}$, $R^{4'}$ and $R^{5'}$ into the radicals $R^1$, $R^{1a}$, $R^4$ and $R^5$ which have the meanings given in claims 1 to 4.

**6.** The compound of the formula I as claimed in one or more of claims 1 to 4 and/or its physiologically acceptable salts for use as a pharmaceutical.

**7.** A pharmaceutical preparation, which comprises one or more compounds of the formula I as claimed in one or more of claims 1 to 4 and/or its physiologically acceptable salts in addition to pharmaceutically acceptable carriers and/or additives.

**8.** The use of compounds of the formula I as claimed in one or more. of claims 1 to 4 and/or their physiologically acceptable salts for preparing a medicament for the activation of soluble guanylate cyclase.

**9.** The use of compounds of the formula I as claimed in one or more of claims 1 to 4 and/or their physiologically acceptable salts for preparing a medicament for the therapy or prophylaxis of cardiovascular diseases, endothelial dysfunction, diastolic dysfunction, atherosclerosis, hypertension, angina pectoris, thromboses, restenoses, myocardial infarction, strokes, cardiac insufficiency, pulmonary hypertonia, erectile dysfunction, asthma bronchiale, chronic kidney insufficiency, diabetes or cirrhosis of the liver or for improving memory performance or a restricted ability to learn.

**Revendications**

**1.** Composés de formule 1

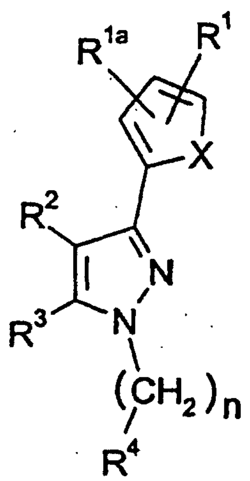

I

dans laquelle
X représente O, S, NH ou $N(CH_3)$;
$R^1$ représente $CO-NR^{11}R^{12}$;
$R^{1a}$ représente un atome d'hydrogène;
$R^2$ et $R^3$ représentent, indépendamment l'un de l'autre, un atome d'hydrogène, un reste alkyle en $C_1$-$C_{10}$, aryle en $C_6$-$C_{14}$, (aryl en $C_6$-$C_{14}$)-(alkyle en $C_1$-$C_4$), cycloalkyle en $C_3$-$C_7$, (cycloalkyl en $C_3$-$C_7$)-(alkyle en $C_1$-$C_4$), le reste Het ou le reste Het-(alkyle en $C_1$-$C_4$), les restes alkyle, les restes aryle, les restes arylalkyle, les restes cycloalkyle, les restes cycloalkylalkyle, les restes Het et les restes Het-alkyle représentés par $R^2$ ou $R^3$ pouvant être à chaque fois non substitués ou substitués par un ou plusieurs substituants $R^5$,
ou $R^2$ et $R^3$ forment ensemble avec les atomes de carbone qui les portent un cycle carbocyclique de 5 à 7 chaînons pouvant contenir une ou plusieurs doubles liaisons et pouvant être non substitué ou substitué par un ou plusieurs substituants $R^5$;
$R^4$ représente un atome d'hydrogène, un reste alkyle en $C_1$-$C_{10}$, aryle en $C_6$-$C_{14}$, (aryl en $C_6$-$C_{14}$)-(alkyle en $C_1$-$C_4$), cycloalkyle en $C_3$-$C_7$, (cycloalkyl en $C_3$-$C_7$)-(alkyle en $C_1$-$C_4$), le reste Het ou le reste Het-(alkyle en $C_1$-$C_4$), les restes alkyle, les restes aryle, les restes arylalkyle, les restes cycloalkyle, les restes cycloalkylalkyle, les restes Het et les restes Het-alkyle représentés par $R^4$ pouvant être à chaque fois non substitués ou substitués par un ou plusieurs substituants $R^5$, et, lorsque n = 0, $R^4$ ne peut pas être un atome d'hydrogène;
n est 0, 1 ou 2;
Het représente un hétérocycle de 5 à 7 chaînons, saturé ou insaturé;
$R^5$ représente un reste halogéno, alkyle en $C_1$-$C_5$, $OR^6$, $NR^7R^8$, $CO-OR^9$, $CO-R^{10}$, $CO-NR^{11}R^{12}$, $CO-NR^{12}-OR^{11}$, $S(O)_m-R^{13}$, $S(O)_2-NR^{14}R^{15}$, $NO_2$, CN ou $CF_3$, et des restes $R^5$ apparaissant plusieurs fois sont indépendants les uns des autres et peuvent être identiques ou différents;
$R^6$, $R^7$, $R^8$, $R^{11}$ et $R^{14}$ représentent, indépendamment les uns des autres, un atome d'hydrogène, un reste alkyle en $C_1$-$C_{10}$, aryle en $C_6$-$C_{14}$, (aryl en $C_6$-$C_{14}$)-(alkyle en $C_1$-$C_4$), cycloalkyle en $C_3$-$C_7$, (cycloalkyl en $C_3$-$C_7$)-(alkyle en $C_1$-$C_4$), le reste Het, le reste Het-(alkyle en $C_1$-$C_4$), $CO-R^{16}$ ou $S(O)_2-R^{17}$, les restes alkyle, les restes aryle, les restes arylalkyle, les restes cycloalkyle, les restes cycloalkylalkyle, les restes Het et les restes Het-alkyle représentés par $R^6$, $R^7$, $R^8$, $R^{11}$ et $R^{14}$ pouvant être à chaque fois non substitués ou substitués par un ou plusieurs substituants identiques ou différents de la série des restes halogéno, alkyle en $C_1$-$C_5$, $OR^{18}$, $NR^{19}R^{20}$, $CO-OR^{21}$, $CO-R^{22}$, $CO-NR^{23}R^{24}$, $CO-NR^{24}-OR^{23}$, $S(O)_m-R^{25}$, $S(O)_2-NR^{26}R^{27}$, $NO_2$, CN et $CF_3$, et des restes $R^6$, $R^7$, $R^8$, $R^{11}$ et $R^{14}$ apparaissant plusieurs fois sont indépendants les uns des autres et peuvent être identiques ou différents;
$R^9$, $R^{10}$, $R^{12}$, $R^{13}$ et $R^{15}$ représentent, indépendamment les uns des autres, un atome d'hydrogène, un reste alkyle en $C_1$-$C_{10}$, aryle en $C_6$-$C_{14}$, (aryl en $C_6$-$C_{14}$)-(alkyle en $C_1$-$C_4$), cycloalkyle en $C_3$-$C_7$, (cycloalkyl en $C_3$-$C_7$)-(alkyle en $C_1$-$C_4$), le reste Het ou le reste Het-(alkyle en $C_1$-$C_4$), les restes alkyle, les restes aryle, les restes arylalkyle, les restes cycloalkyle, les restes cycloalkylalkyle, les restes Het et les restes Het-alkyle représentés par $R^9$, $R^{10}$, $R^{12}$, $R^{13}$ et $R^{15}$ pouvant être à chaque fois non substitués ou substitués par un ou plusieurs substituants identiques ou différents de la série des restes halogéno, alkyle en $C_1$-$C_5$, $OR^{18}$, $NR^{19}R^{20}$, $CO-OR^{21}$, $CO-R^{22}$, $CO-NR^{23}R^{24}$, $CO-NR^{24}-OR^{23}$, $S(O)_m-R^{25}$, $S(O)_2-NR^{26}R^{27}$, $NO_2$, CN et $CF_3$, et des restes $R^9$, $R^{10}$, $R^{12}$, $R^{13}$ et $R^{15}$ apparaissant plusieurs fois sont indépendants les uns des autres et peuvent être identiques ou différents;
ou les deux restes $R^7$ et $R^8$, les deux restes $R^{11}$ et $R^{12}$ et les deux restes $R^{14}$ et $R^{15}$, à chaque fois avec l'atome

d'azote portant les deux restes, forment un cycle hétérocyclique de 5 à 7 chaînons, saturé ou insaturé, qui peut contenir un hétéroatome cyclique supplémentaire de la série de l'azote, de l'oxygène et du soufre et qui peut être substitué par un ou plusieurs substituants identiques ou différents de la série des restes alkyle en $C_1$-$C_4$ et des halogènes;

$R^{16}$ représente un atome d'hydrogène, un reste alkyle en $C_1$-$C_6$, cycloalkyle en $C_3$-$C_7$, (cycloalkyl en $C_3$-$C_7$)-(alkyle en $C_1$-$C_4$), aryle en $C_6$-$C_{14}$, (aryl en $C_6$-$C_{14}$)-(alkyle en $C_1$-$C_4$), le reste Het ou le reste Het-(alkyle en $C_1$-$C_4$);

$R^{17}$ représente un reste alkyle en $C_1$-$C_6$, cycloalkyle en $C_3$-$C_7$, (cycloalkyl en $C_3$-$C_7$)-(alkyle en $C_1$-$C_4$), aryle en $C_6$-$C_{14}$, (aryl en $C_6$-$C_{14}$)-(alkyle en $C_1$-$C_4$), le reste Het ou le reste Het-(alkyle en $C_1$-$C_4$);

$R^{18}$, $R^{19}$, $R^{20}$, $R^{23}$ et $R^{26}$ représentent, indépendamment les uns des autres, un atome d'hydrogène, un reste alkyle en $C_1$-$C_{10}$, cycloalkyle en $C_3$-$C_7$, (cycloalkyl en $C_3$-$C_7$)-(alkyle en $C_1$-$C_4$), aryle en $C_6$-$C_{14}$, (aryl en $C_6$-$C_{14}$) -(alkyle en $C_1$-$C_4$), le reste Het, le reste Het-(alkyle en $C_1$-$C_4$), CO-$R^{16}$ ou $S(O)_2$-$R^{17}$, et des restes $R^{18}$, $R^{19}$, $R^{20}$, $R^{23}$ et $R^{26}$ apparaissant plusieurs fois sont indépendants les uns des autres et peuvent être identiques ou différents;

$R^{21}$, $R^{22}$, $R^{24}$, $R^{25}$ et $R^{27}$ représentent, indépendamment les uns des autres, un atome d'hydrogène, un reste alkyle en $C_1$-$C_{10}$, cycloalkyle en $C_3$-$C_7$, (cycloalkyl en $C_3$-$C_7$)-(alkyle en $C_1$-$C_4$), aryle en $C_6$-$C_{14}$, (aryl en $C_6$-$C_{14}$) -(alkyle en $C_1$-$C_4$), le reste Het ou le reste Het-(alkyle en $C_1$-$C_4$), et des restes $R^{21}$, $R^{22}$, $R^{24}$, $R^{25}$ et $R^{27}$ apparaissant plusieurs fois sont indépendants les uns des autres et peuvent être identiques ou différents;

ou les deux restes $R^{19}$ et $R^{20}$, les deux restes $R^{23}$ et $R^{24}$ et les deux restes $R^{26}$ et $R^{27}$, à chaque fois avec l'atome d'azote portant les deux restes, forment un cycle hétérocyclique de 5 à 7 chaînons, saturé ou insaturé, qui peut contenir un hétéroatome cyclique supplémentaire de la série de l'azote, de l'oxygène et du soufre et qui peut être substitué par un ou plusieurs substituants identiques ou différents de la série des restes alkyle en $C_1$-$C_4$ et des halogènes;

m est 0, 1 ou 2;

sous toutes leurs formes stéréo-isomères et leurs mélanges en toutes proportions, et leurs sels physiologiquement acceptables.

**2.** Composés de formule I selon la revendication 1, dans lesquels $R^2$ et $R^3$ forment ensemble, avec les atomes de carbone qui les portent, un cycle benzène pouvant être non substitué ou substitué par un ou plusieurs substituants $R^5$ identiques ou différents, sous toutes leurs formes stéréo-isomères et leurs mélanges en toutes proportions, et leurs sels physiologiquement acceptables.

**3.** Composés de formule I selon la revendication 1 et/ou 2, dans lesquels X représente O ou S, sous toutes leurs formes stéréo-isomères et leurs mélanges en toutes proportions, et leurs sels physiologiquement acceptables.

**4.** Composés de formule I selon l'une ou plusieurs des revendications 1 à 3, dans lesquels $R^{11}$ représente un atome d'hydrogène et $R^{12}$ un reste alkyle en $C_1$-$C_4$ non substitué, alkyle en $C_1$-$C_4$ substitué par un reste de la série de $OR^{18}$, $NR^{19}R^{20}$ et CO-$NR^{23}R^{24}$, hétéroaryle de 5 ou 6 chaînons, phényle non substitué ou phényle substitué par un, deux ou trois restes identiques ou différents de la série des restes halogéno, alkyle en $C_1$-$C_5$, $OR^{18}$, $NR^{19}R^{20}$, CO-$OR^{21}$, CO-$R^{22}$, CO-$NR^{23}R^{24}$, $S(O)_m$-$R^{25}$, $S(O)_2$-$NR^{26}R^{27}$, $NO_2$, CN et $CF_3$, sous toutes leurs formes stéréo-isomères et leurs mélanges en toutes proportions, et leurs sels physiologiquement acceptables.

**5.** Procédé de préparation de composés de formule I selon l'une ou plusieurs des revendications 1 à 4, **caractérisé en ce que**

a) on fait réagir des composés 1,3-dicarbonyle de formule II avec des hydrazines de formule III ou leurs sels

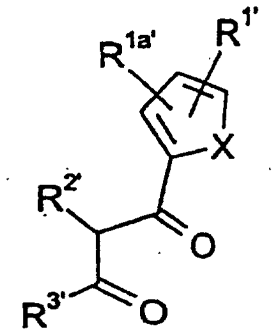

II

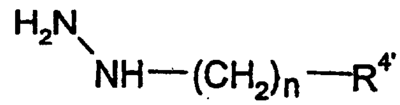

III

où, dans les formules II et III, les restes X, $R^{1'}$, $R^{1a'}$ et $R^{4'}$ et le nombre n ont la signification donnée dans les revendications 1 à 4 pour X, $R^1$, $R^{1a}$, $R^4$ et n, et les restes $R^{2'}$ et $R^{3'}$ ont la signification donnée dans les revendications 1 à 4 pour $R^2$ et $R^3$, mais ne peuvent pas former ensemble de cycle aromatique avec les atomes de carbone qui les portent, et les groupes fonctionnels des restes $R^{1'}$, $R^{1a'}$, $R^{2'}$, $R^{3'}$ et $R^{4'}$ peuvent en outre se trouver sous forme protégée ou sous forme de précurseurs, puis on transforme éventuellement les restes $R^{1'}$, $R^{1a'}$, $R^{2'}$, $R^{3'}$ et $R^{4'}$ en les restes $R^1$, $R^{1a}$, $R^2$, $R^3$ et $R^4$ ayant la signification donnée dans les revendications 1 à 4; ou

b) on fait réagir des composés de formule VII avec des hydrazines de formule III ou leurs sels

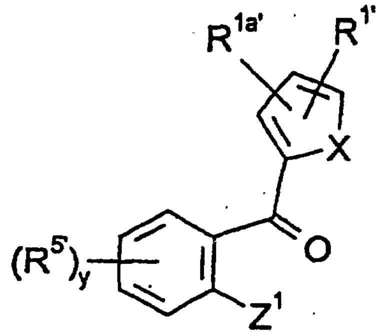

VII

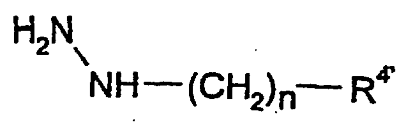

III

où, dans les formules VII et III, les restes X, $R^{1'}$, $R^{1a'}$ et $R^{4'}$ et le nombre n ont la signification donnée dans a), $R^{5'}$ a la signification donnée pour $R^5$ dans les revendications 1 à 4, et les groupes fonctionnels des restes $R^{5'}$ peuvent en outre se trouver sous forme protégée ou sous forme de précurseurs, y est 0, 1, 2, 3 ou 4 et $Z^1$ représente un groupe partant, puis on transforme éventuellement les restes $R^{1'}$, $R^{1a'}$, $R^{4'}$ et $R^{5'}$ en les restes $R^1$, $R^{1a}$, $R^4$ et $R^5$ ayant la signification donnée dans les revendications 1 à 4; ou

c) on fait réagir des composés de formule VII avec des composés de formule IX avec élimination du groupe acyle R-CO pour obtenir des composés de formule X, que l'on alkyle avec des composés de formule XI

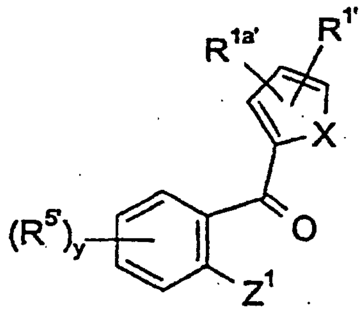

VII

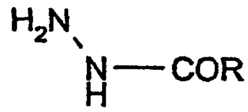

IX

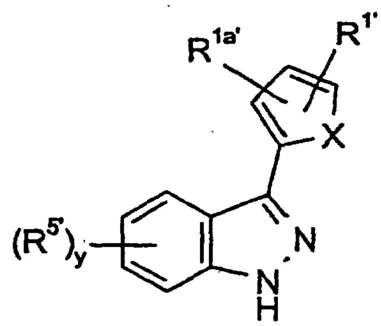

X

$$R^{4'}-(CH_2)_n-Z^2$$

XI

où, dans les formules VII, IX, X et XI, les restes X, $R^{1'}$, $R^{1a'}$, $R^{4'}$ et $R^{5'}$, $Z^1$, n et y ont la signification donnée dans a) et b), $Z^2$ représente un groupe partant et R représente un reste alkyle ou un reste aryle, puis on transforme éventuellement les restes $R^{1'}$, $R^{1a'}$, $R^{4'}$ et $R^{5'}$ en les restes $R^1$, $R^{1a}$, $R^4$ et $R^5$ ayant la signification donnée dans les revendications 1 à 4.

**6.** Composés de formule I selon l'une ou plusieurs des revendications 1 à 4 et/ou leurs sels physiologiquement acceptables à utiliser comme médicaments.

**7.** Composition pharmaceutique, **caractérisée en ce qu'**elle contient un ou plusieurs composés de formule I selon l'une ou plusieurs des revendications 1 à 4 et/ou leurs sels physiologiquement acceptables, avec des supports et/ou des additifs pharmaceutiquement acceptables.

**8.** Utilisation de composés de formule I selon l'une ou plusieurs des revendications 1 à 4 et/ou de leurs sels physiologiquement acceptables pour la préparation d'un médicament destiné à activer la guanylate-cyclase soluble.

**9.** Utilisation de composés de formule I selon l'une ou plusieurs des revendications 1 à 4 et/ou de leurs sels physiologiquement acceptables pour la préparation d'un médicament destiné à la thérapie ou à la prophylaxie de maladies cardio-vasculaires, du dysfonctionnement endothélial, du dysfonctionnement diastolique, de l'athérosclérose, de l'hypertension artérielle, de l'angine de poitrine, de thromboses, de resténoses, de l'infarctus du myocarde, des attaques d'apoplexie, de l'insuffisance cardiaque, de l'hypertonie pulmonaire, du dysfonctionnement érectile, de l'asthme bronchique, de l'insuffisance rénale chronique, du diabète ou de la cirrhose du foie, ou pour améliorer une capacité à apprendre ou une performance de la mémoire limitées.